# EUROPEAN PATENT SPECIFICATION

(11) **EP 0 678 517 B1**
(45) Date of publication and mention of the grant of the patent: **03.07.2002**
(21) Application number: 94302758.1
(22) Date of filing: 19.04.1994
(51) Int. Cl.: C07D 311/96, C07D 491/107, C07D 409/04, G03C 1/685, C07D 495/10, C07D 493/10

(54) **Spiropyrone compounds**
Spiropyrone Derivate
Dérivés de spiropyrones

(43) Date of publication of application: 25.10.1995
(73) Proprietor: TOKUYAMA CORPORATION, Tokuyama-shi Yamaguchi-ken (JP)
(72) Inventor: Momoda, Junji, Tokuyama-shi, Yamaguchi-ken (JP); Imura, Satoshi, Tokuyama-shi, Yamaguchi-ken (JP); Kobayakawa, Takashi, Tokuyama-shi, Yamaguchi-ken (JP)
(74) Representative: Cresswell, Thomas Anthony

(56) References cited:
- EP-A- 0 246 114
- EP-A- 0 362 771
- EP-A- 0 401 958
- US-A- 4 980 089

## Description

The present invention relates spiropyrone compounds and to processes for preparing spiropyrone compounds. More specifically, the invention relates to a process for obtaining in good yields spiropyrone compounds that are starting materials of spiropyran compounds which change from a colorless state into a colored or a densely colored state upon irradiation with the light containing ultraviolet rays such as the sunlight or the light of a mercury lamp, the change being reversible, and maintaining excellent durability.

The photochromism is a phenomenon which is drawing attention in the past several years, and is a reversible action of a compound which quickly changes the color when it is irradiated with the light containing ultraviolet rays such as the sunlight or the light or a mercury lamp, and returns to the initial color when it is placed in a dark place without being irradiated with the light. The compounds having such properties are called photochromic compounds. A variety of compounds have heretofore been synthesized without, however, having any particular skeleton which is common in the structures thereof.

Japanese Laid-Open Patent Publication No. 11075/1991 discloses, as photochromic compounds, spiropyran compounds having a bicyclo[3,3,1]9-nonylidene group bonded to the second position or a benzopyran ring or the naphthopyran ring. These spiropyran compounds have excellent photochromic properties changing from a colorless state into a state of developing yellow to orange color upon the irradiation with ultraviolet rays at normal temperature (10 to 40°C) or in a temperature range slightly higher than-the normal temperature, returning from the colored state back to the colorless state within short periods of time, and exhibiting good color density. These spiropyran compounds are obtained by first obtaining as a starting material, a spiropyrone compound in which a bicyclo[3,3,1]9-nonylidene group is bonded to the second position of a benzopyron ring or a naphthopyron ring by reacting a 1-acetyl-2-naphthol, a 1-acetyl-2-benzole or the like with a bicyclo[3,3,1]nonane-9-one or the like.

US-A-4980089 discloses a series of benzospiropyran and of naphthospiropyran compounds.

EP-A-0246114 discloses a series of photochromic spiropyrans.

EP-A-0362771 discloses photochromic spiropyrans.

### Summary of the Invention

In the process for preparing the spiropyran compounds, however, the conversion for synthesizing the spiropyrone compounds is not so high that the desired spiropyran compounds are not obtained maintaining a satisfactory yield.

Under such circumstances, it has been desired to develop a process capable of obtaining in good yields spiropyrone compounds that serve as starting materials for preparing spiropyran compounds having excellent photochromic properties.

In view of the above-mentioned problems, the present inventors have conducted keen study, and have discovered the fact that the above problems could be solved by offering the structure in which a 2-bicyclo[3,3,1]9-nonenylidene group is bonded to the second position of a benzopyrone ring or the naphthopyrone ring of the spiropyrone compound, and have thus arrived at the present invention.

That is, the present invention provides a spiropyrone compound of formula (III), 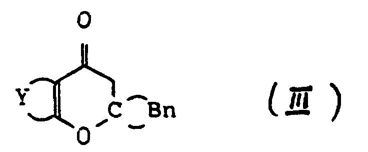 wherein 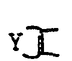 is a substituted or an unsubstituted aromatic hydrocarbon group or a substituted or an unsubstituted unsaturated heterocyclic group, and 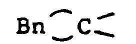 is a substituted or an unsubstituted 2-bicyclo-[3,3,1]-9-nonenylidene group.

The invention also provides a process for preparing such a spiropyrone compound, which comprises reacting a compound of formula (I), 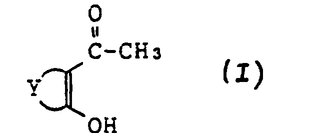 wherein 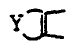 is as defined above,
with a compound represented by the following general formula (II), 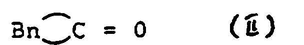 wherein 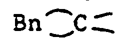 is as defined above.

### Brief Description of the Drawings

Fig. 1 is a chart of nuclear magnetic resonance spectra of protons of a spiropyrone compound obtained in Example 1; and
Fig. 2 is a chart of nuclear magnetic resonance spectra of protons of a spiropyran compound obtained in Application Example 1.

### Detailed Description of the Invention

When a compound represented by the above-mentioned general formula (I) is reacted with a compound represented by the above-mentioned general formula (II), the group 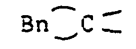 which is a 2-bicyclo[3,3,1]9-nonenylidene group or a substitutent thereof of the compound of the general formula (II) works to increase the reactivity. According to the present invention, therefore, it is made possible to obtain a spiropyrone compound represented by the above-mentioned general formula (III) maintaining a high yield.

According to the present invention, any known compound represented by the above-mentioned general formula (I) can be used without any limitation. In the compound of the above general formula (I), a group represented by 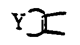 is a substituted or an unsubstituted aromatic hydrocarbon group or a substituted or an unsubstituted unsaturated heterocyclic group. A concrete example of the aromatic hydrocarbon group is a divalent group having 6 to 14 carbon atoms, and a divalent group derived from one benzene ring or a fused ring comprising 2 to 4 benzene rings is especially preferable. As examples of the ring constituting the aromatic hydrocarbon group, there can be mentioned a benzene ring, a naphthalene ring and a phenantherne ring. The aromatic hydrocarbon group may be substituted at least one member, or preferably, with 1 to 3 members. As example of aromatic hydrocarbon groups substituted with a substituents, there can be mentioned divalent groups derived from aromatic hydrocarbon rings such as toluene, xylene or the like substituted with an alkyl group having 1 to 4 carbon atoms such as a methyl group, an ethyl group or a t-butyl group. In addition to the above-mentioned substituents, there can be mentioned halogen atoms such as fluorine, chlorine, bromine and the like; a hydroxyl group; a cyano group; a nitro group; alkoxy groups having 1 to 4 carbon atoms such as a methoxy group, an ethoxy group and a tert-butoxy group; aryl groups having 6 to 10 carbon atoms such as a phenyl group and a tolyl group; substituted amino groups such as an alkylamino group having to 4 carbon atoms like a methylamino group and an ethnylamino group, and a dialkylamino group having 2 to 8 carbon atoms like a dimethylamino group and a diethylamino group; an alkyl group substituted with halogen having 1 to 2 carbon atoms such as a trifluoromethyl group; 5-membered and 6-membered monocyclic heterocyclic groups having 1 or 2 oxygen atoms, sulfur atoms or nitrogen atoms, such as a thienyl group, a furyl group or a pyrrolyl group. In the polysubstitution product substituted with these substituents, the substituents may be the same or different, and the positions of the substituents are changed depending upon the object and the application.

As the unsaturated heterocyclic group 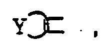 there can be mentioned divalent group having 4 to 10 carbon atoms, and a 5- or 6-membered monocylic hetrocyclic group containing one or two of nitrogen, oxygen and sulfur atoms, or a fused heterocyclic group formed by fusing a benzene ring to the above-mentioned monocyclic heterocyclic group. As the ring constituting the unsaturated heterocyclic group, there can be mentioned nitrogen-containing rings such as a pyridine ring, a quinoline ring and a pyrrole ring, oxygen-containing rings such as a furan ring and a benzofuran ring, and sulfur-containing rings such as a thiophene ring and a benzothiophene ring. According to the present invention, furthermore, it is allowable to use without any limitation the substituted and unsaturated heterocyclic groups in which the substituents described in relation to the above aromatic hydrocarbon groups are substituted for the above unsaturated heterocyclic rings.

According to the present invention, any known compound represented by the above-mentioned general formula (II) can be used without any limitation. In the compound of the general formula (II), the group 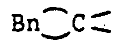 is a 2-bicyclo[3,3,1]9-nonenylidene group 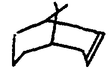 or a 2-bicyclo[3,3,1]9-nonenylidene group substituted with a substituent. Concrete examples of the substituent include halogen atoms such as fluorine, chlorine and bromine; a hydroxyl group; a cyano group; a nitro group, a carboxyl group; alkyl groups having 1 to 4 carbon atoms such as a methyl group, an ethyl group and a t-butyl group; alkoxy groups having 1 to 4 carbon atoms such as a methoxy group, an ethoxy group and a tert-butoxy group; alkyl groups substituted with halogen having 1 or 2 carbon atoms such as a trifluoromethyl group and the like; aryl groups having 6 to 10 carbon atoms such as a phenyl group and a tolyl group; aryloxy groups having 6 to 10 carbon atoms such as a phenoxy group and a 1-naphthoxy group; aralkyl groups having 7 to 10 carbon atoms such as a benzyl group, a phenylethyl group and a phenylpropyl group; aralkoxy groups having 7 to 10 carbon atoms such as a benzyloxy group and the like; substituted amino groups such as an alkylamino group having 1 to 4 carbon atoms like a methylamino group and an ethylamino group, and a dialkylamino group having 2 to 8 carbon atoms like a dimethylamino group and diethylamino group; and alkoxycarbonyl groups having 2 to 10 carbon atoms such as an ethoxycarbonyl group and the like. These substituents are included not only as a one-substitution product but may also be included as a substitution product having two or more and, preferably, having 1 to 3 substituents. In the polysubstitution product, the substituents may be the same or different, and the positions of the substituents are changed depending upon the object and application.

According to the present invention, the reaction of the compound represented by the general formula (I) with the compound represented by the general formula (II) can be carried out under any condition without any particular limitation. Here, the reaction ratio of the mixture of these two compounds can be selected to lie over a wide range, and is usually selected to lie over a range of from 1:10 to 10:1 (molar ratio). The reaction temperature should usually be from 0 to 200°C, and the reaction time is usually selected to be from 1 to 20 hours. As a solvent for the above reaction, there can be used a polar non-protonic solvent such as an N-methylpyrolidone, a dimethylformamide, a toluene, a benzene, a tetrahydrofurane and the like.

The reaction is usually carried out in the presence of a condensing agent. Though there is no particular limitation, the condensing agent is generally a primary amine or a secondary amine. Concretely speaking, the primary amine or the secondary amine should be the one represented by the general formula, 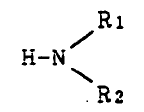 or 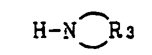 where either one of the groups R₁ and R₂ is a hydrogen atom and the other one is an alkyl group, or both of them are the same or different alkyl groups.

Though there is no particular limitation, the alkyl group should generally have 1 to 6 carbon atoms. Concrete examples include a methyl group, an ethyl group, a propyl group and a butyl group. Here, preferred examples of the primary amine include an N-ethylamine, an N-propylamine and the like. Furthermore, R₃ should be an alkylene group having 3 to 6 carbon atoms such as a tetramethylene group or a pentamethylene group; an oxyalkylene group having 3 to 6 carbon atoms such as 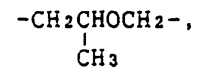 -CH₂OCH₂CH₂-, -CH₂CH₂OCH₂-, or -CH₂O(CH₂)₃-; a chioalkylene group having 3 to 6 carbon atoms such as -CH₂SCH₂CH₂-, -CH₂S(CH₂)₃-, or -CH₂CH₂SCH₂CH₂-; or an azoalkylene group having 3 to 6 carbon atoms such as 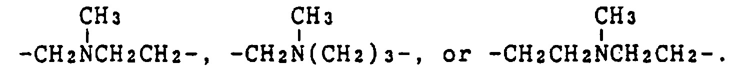 Here, preferred examples of the secondary amine include a diethylamine, a pyrrolydine, a piperidine, and a morpholine. The condensing agents should be used in an amount of, usually, from 0.1 to 10 moles per mole of the compound represented by the general formula (I).

The above-mentioned reaction is completed by removing the water formed during the reaction. The water can be removed either based on the azeotropy by using the Dien-Stark's device to remove the water out of the reaction system or by adding a dehydrating agent such as a calcium chloride, a calcium oxide or a zinc chloride to the reaction system to remove the water forming in the system.

Here, when the reaction of the compound represented by the general formula (I) with the compound represented by the general formula (II) is excessively continued in the presence of the primary amine or the secondary amine as the condensing agent, the spiropyrone compound represented by the general formula (III) that is once formed further reacts with the primary amine or the secondary amine, and a spiropyran compound represented by the following general formula (V) is gradually formed, 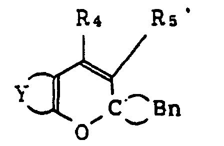 wherein 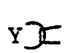 is a substituted or an unsubstituted aromatic hydrocarbon group or a substituted or an unsubstituted unsaturated heterocyclic group, 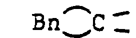 is a substituted or an unsubstituted 2-bicyclo[3,3,1]9-nonenylidene group, and either one of R₄ and R₅' is a substituted amino group and the other is a hydrogen atom. However, that spiropyran compound easily returns back to the spiropyrone compound represented by the general formula (III). In such a case, therefore, the reaction solution obtained by the reaction of the compound represented by the general formula (I) with the compound represented by the general formula (II) should be mixed with an aqueous solution of the acid, in order to return the above formed spiropyran compound back again to the spiropyrone compound represented by the general formula (III). In this case, though there is no particular limitation, the acid should usually be a mineral acid such as hydrochloric acid or sulfuric acid. Though there is no particular limitation, the acid is used in an amount equivalent to, or greater than, the amount of the condensing agent so that the reaction system becomes acidic. Water should be contained in an amount equal to, or greater than, the amount of the compound represented by the general formula (I) that is fed into the reaction solution. The aqueous solution of the acid may be added by preparing the aqueous solution of the acid in advance and mixing it into the reaction solution, or by separately mixing the acid and the water.

The spiropyrone compound represented by the general formula (III) obtained through the above-mentioned reaction can be separated and refined by any method but, usually, through the recrystallization.

The reaction of the compound represented by the general formula (I) with the compound represented by the general formula (II) forms the spiropyrone compound represented by the general formula (III), 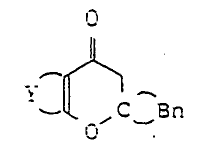 wherein 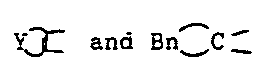 are the same as those of the general formula (I) and the general formula (II).

Representative examples of the spiropyrone compounds are as follows:
1) Spiro(2-bicyclo[3,3,1]nonene-9,2'-(2H)benzo(h)-4'-chromanone)
2) Spiro(2-bicyclo[3,3,1]nonene-9,2'-(2H)benzo(f)-4'-chromanone)
3) 8'-Cyanospiro(2-bicyclo[3,3,1]nonene-9,2'-2(H)-4'-chromanone)
4) 5'-Methoxy-7'-ethylspiro(2-bicyclo[3,3,1]nonene-9,2'-(2H)-4'-chromanone)
5) 5-Dimethylaminospiro(2-bicyclo[3,3,1]nonene-9,2'-(2H)benzo(f)-4'-chromanone)
6) 8-Methoxy-5'-chlorospiro(2-bicyclo[3,3,1]nonene-9,2'-(2H)benzo(f)-4'-chromanone)
7) Spiro(2-bicyclo[3,3,1]nonene-9,2'-(2H)isoquino(4,3-b)-4'-pyranone)
8) 5,8,8-Trimethylspiro(2-bicyclo[3,3,1]nonene-9,2'-(2H)pyrido(2,3-b)-4'-pyranone)
9) 7'-Phenyl-5-n-propylspiro(2-bicyclo[3,3,1]nonene-9,2'-2(H)benzo(h)-4'-chromanone)
10) 7'-Phenyl-5-chlorospiro(2-bicyclo[3,3,1]nonene-9,2'-(2H)-4'-chromanone)
11) Spiro(2-bicyclo[3,3,1]nonene-9,2'-(2H)pyrido(3,2-f)-4'-chromanone)
12) 5,7,7-Trimethylspiro(2-bicyclo[3,3,1]nonene9,2'-(2H)dibenzo(f,h)-4'-chromanone)
13) 7'-Nitro-10'-ethoxy-5,7,7-trimethylspiro(2-bicyclo[3,3,1]nonene-9,2'-(2H)benzo(h)-4'-chromanone)
14) 7'-Trifluoromethyl-10'-n-pentyl-5,7,7-trimethylspiro(2-bicyclo[3,3,1]nonene-9,2'-(2H)benzo(h)-4'-chromanone)
15) 5,10'-Diphenylspiro(2-bicyclo[3,3,1]nonene-9,2'-(2H)benzo(f)-4'-chromanone)
16) 7'-Thienylspiro(2-bicyclo[3,3,1]nonene-9,2'-(2H)benzo(h)-4'-chromanone)
17) 5-Trifluoromethyl-10'-n-propoxyspiro(2-bicyclo[3,3,1]-9,2'-(2H)pyrido(2,3-h)-4'-chromanone)
18) 5-Cyano-7'-furanylspiro(2-bicyclo[3,3,1]nonene-9,2'-(2H)benzo(h)-4'-chromanone)
19) 5-Benzyl-7'-dimethylaminospiro(2-bicyclo[3,3,1]nonene-9,2'-(2H)benzo-4'-chromanone)
20) 5-n Butyl 7-fluoro-6'-bromo-8'-hydroxyspiro(2-bicyclo[3,3,1]nonene-9,2'-(2H)benzo(f)-4'-chromanone)
21) 5-Ethyl-1-ethoxyspiro(2-bicyclo[3,3,1]nonene-9,2'-(2H)(benzo(b)thieno(2,3-b)-4'-pyranone)
22) Spiro(2-bicyclo[3,3,1]nonene-9,2'-(2H)methylpyrrolo(2,3-b)-4'-pyranone)
23) 5-Chlorospiro(2-bicyclo[3,3,1]nonene-9,2'-(2H)quino(3,4-b)-4'-pyranone)
24) 7-Bromospiro(2-bicyclo[3,3,1]nonene-9,2'-(2H)fluoro(2,3-b)-4'-pyranone)
25) 5'-Bromospiro(2-bicyclo[3,3,1]nonene-9,2'-2(H)thieno(2,3-b)-4'-pyranone)

The spiropyrone compounds obtained according to the present invention generally exist in the form of colorless and viscous liquids at normal temperature under normal pressure, and can be confirmed by means described in (a) to (c) below.
(a) The kind and number of protons existing in the molecules can be learned by the nuclear magnetic resonance spectrum of protons (H¹-NMR). That is, a peak appears near δ7 to 8.5 ppm due to aromatic protons, and broad peaks appear near δ1.2 to 3.5 ppm and near 5.3 to 5.7 ppm due to protons of a 2-bicyclo[3,3,1]9-nonenylidene group and protons at the third position of a spiropyrone ring. Further, the number of protons in the bonding group can be learned by comparing their δ peak intensities.
(b) Amounts in percent by weight of carbon, hydrogen, nitrogen, sulfur and halogen can be found by the elemental analysis. The amount in percent by weight of oxygen can be calculated by subtracting the sum of amounts in percent by weight of the above-found elements from 100. Therefore, the composition of the formed product can be determined.
(c) The kind of carbon present in the molecules can be learned by the 13C-nuclear magnetic resonance spectrum (¹³C- NMR). Peaks appear near δ27 to 52 ppm due to carbon of the 2-bicyclo[3,3,1]9-nonenylidene group and carbon at the third position of the spiropyrone ring, and a peak appears near δ 110 to 150, ppm due to carbon of an aromatic hydrocarbon group or an unsaturated heterocyclic group.

Next, the spiropyrone compound obtained by the above-mentioned process can be transformed into a spiropyran compound having good photochromic properties by changing the α-pyrone ring into a γ-pyran. These spiropyran compounds are of formula (IV) 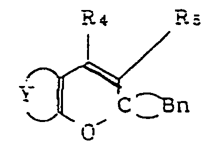 wherein 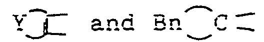 are the same as those of the aforementioned general formulas (I) and (II), and R₄ and R₅ are the same or different hydrogen atoms, alkyl groups, aralkyl groups, aryl groups or substituted amino groups, respectively, and when either one of R₄ and R₅ is a substituted amino group, the other one is a hydrogen atom.

The present invention provides processes (a) to (e), set out below, for preparing spiropyran compounds of formula (IV).

In the above-mentioned spiropyran compound, though there is no particular limitation, the alkyl group should generally be the one having 1 to 20 carbon atoms and, preferably, 1 to 6 carbon atoms. The aralkyl group and the alkyl group should generally have 1 to 10 carbon atoms and, preferably, 1 to 4 carbon atoms. Concrete examples of the alkyl group and the aralkyl group include a methyl group, an ethyl group, a propyl group, a butyl group, a benzyl group, a phenylethyl group, a phenylpropyl group and a phenylbutyl group. The aryl group should have 6 to 10 carbon atoms. Preferred examples thereof include a phenyl group, a tolyl group, a xylyl group and a naphthyl group.

Furthermore, the substituted amino groups represented by R₄ and R₅ in the above general formula (IV) are the ones represented by the general formula 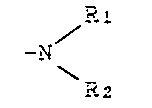 wherein R₁ and R₂ are as defined above, or 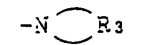 wherein R₃ is as defined above, which are residual groups remaining after hydrogen atoms are removed from the primary amine or the secondary amine used as the condensing agent.

Examples of the spiropyran compound represented by the above general formula (IV) are as follows:
1) Spiro(2-bicyclo(3,3,1)nonene-9,2'-(2H)benzo(h)chromene)
2) spiro(2-bicyclo(3,3,1)nonene-9,2'-(2H)benzo(f)chromene)
3) 4'-Methylspiro(2-bicyclo(3,3,1)nonene-9,2'-(2H)benzo(f)chromene)
4) 4'-Morpholinospiro(2-bicyclo[3,3,1]nonene-9,2'-(2H)benzo(f)chromene
5) 3'-Methylspiro(2-bicyclo(3,3,1)nonene-9,2'-(2H)benzo(f)chromene)
6) 3',4'-Dimethylspiro(2-bicyclo[3,3,1]nonene-9,2'-(2H)benzo(f)chromene)
7) 8'-Cyano-4'-pyrrolidinospiro(2-bicyclo(3,3,1)nonene-9,2'-(2H)benzochromene)
8) 7'-Ethyl-5'-methoxy-4'-morpholinospiro(2-bicyclo(3,3,1)nonene-9,2'-(2H)benzochromene)
9) 4'-Diethylamino-5-dimethylaminospiro(2-bicyclo(3,3,1)nonene-9,2'-(2H)benzo(f)chromene)
10) 5'-Chloro-4'-(1,3-thiazolino)-7-methoxyspiro(2-bicyclo(3,3,1)nonene-9,2'-(2H)benzo(f)chromene)
11) 4'-(4-Methylpiperazino)spiro(2-bicyclo(3,3,1)nonene-9,2'-(2H)isoquino(4,3-b)pyran)
12) 5,8,8-Trimethylspiro(2-bicyclo(3,3,1)nonene-9,2'-(2H)pyrido(2,3-b)pyran)
13) 8'-Cyanospiro(2-bicyclo(3,3,1)nonene-9,2'-(2H)chromene)
14) 7'-Phenyl-5-n-propylspiro(2-bicyclo(3,3,1)nonene-9,2'-(2H)benzo(h)chromene)
15) 7'-Phenyl-5-chlorospiro(2-bicyclo(3,3,1)nonone-9,2'-(2H)chromene)
16) Spiro(2-bicyclo(3,3,1)nonone-9,2'-(2H)pyrido(3,2-f)chromene)
17) 5,7,7-trimethylspiro(2-bicyclo(3,3,1)nonone-9,2'-(2H)dibenzo(f,h)chromene)
18) 10'-Ethoxy-5,7,7-trimethyl-7'-nitrospiro(2-bicyclo(3,3,1)nonone-9,2'-(2H)benzo(h)chromene)
19) 5.7.7-Trimethyl-7'-trifluoromethyl-10'-n-pentylspiro(2-bicyclo(3,3,1)nonene-9,2'-(2H)benzo(h)chromene)
20) 5,10'-diphenylspiro(2-bicyclo(3,3,1)nonene-9,2'-(2H)benzo(f)chromene)
21) 7-Thienylspiro(2-bicyclo(3,3,1)nonene-9,2'-(2H)thienylbenzo(h)chromene)
22) 5-Trifluoromethyl-10'-n-propoxyspiro(2-bicyclo(3,3,1)nonene-9,2'-(2H)pyrido(2,3-h)chromene)
23) 5-Cyano-7'-furanylspiro(2-bicyclo(3,3,1)nonene-9,2'-(2H)(benzo(h))chromene)
24) 5-Butyl-7-fluoro-6'-bromo-8'-hydroxyspiro(2-bicyclo(3,3,1)nonene-9,2'-(2H)benzo(f)chromene)
25) 7'-Dimethylamino-5-benzylspiro(2-bicyclo(3,3,1)nonene-9,2'-(2H)benzo(h)chromene)
26) 5-Ethyl-8-ethoxyspiro(2-bicyclo(3,3,1)nonene-9,2'-(2H)(benzo(b)thieno(2,3-b)pyran)
27) Spiro(2-bicyclo(3,3,1)nonene-9,2'-(2H)methylpyrrolo(2,3-b)pyran)
28) 5-Chloro-4'-benzylspiro(2-bicyclo(3,3,1)nonene-9,2'-(2H)quino(3,4-b)pyran)
29) 8'-Cyano-4'-n-butylspiro(2-bicyclo(3,3,1)nonene-9,2'-(2H)chromene)
30) 4'-Phenyl-5,8,8-trimethylspiro(2-bicyclo(3,3,1)nonene-9,2'-(2H)pyrido(2,3-b)pyran
31) 7'-Phenyl-4'-ethyl-5-n-propylspiro(2-bicyclo[3,3,1]nonene-9,2'-(2H)benzo(h)chromene)
32) 4'-n-Butylspiro(2-bicyclo[3,3,1]nonene-9,2'-(2H)benzo(h)chromene)
33) 3'-n-Propylspiro(2-bicyclo[3,3,1]nonene-9,2'-(2H)benzo(f)chromene)
34) 3'-Benzylmethylspiro(2-bicyclo[3,3,1]nonene-9,2'-(2H)benzo(f)chromene)
35) 3'-i-Propylspiro(2-bicycio[3,3,1]nonene-9,2'-(2H)benzo(f)chromene)
36) 3'-p-methoxyphenylspiro(2-bicyclo[3,3,1]nonene-9,2'-(2H)benzo(f)chromene)
37) 3'-Ethylspiro(2-bicyclo[3,3,1]nonene-9,2'-(2H)quino(3,4-b)pyran)
38) 3',4'-Dimethylspiro(2-bicyclo[3,3,1]nonene-9,2'-(2H)fluoro(2,3-b)pyran)
39) 4'-Ethyl-3'-benzyl-5'-bromospiro(2-bicyclo[3,3,1]nonene-9,2'-(2H)thieno(2,3-b)pyran)
40) 3'-n-Propyl-4'-benzylspiro(2-bicyclo[3,3,1]nonene-9,2'-(2H)benzo(f)chromene)
41) 3'-Benzylmethyl-4'-phenylspiro(2-bicyclo[3,3,1]nonene-9,2'-(2H)benzo(f)chromene)
42) 3',4'-Diethylspiro(2-bicyclo[3,3,1]nonene-9',2'-(2H)benzo(h)chromene)

The processes for preparing the spiropyran compound of formula (IV) from the spiropyrone compound of formula (III) are as follows.

In method (c) as set out in the claims, a compound of the general formula (IV) in which either one of R₄ and R₅ is a hydrogen atom and the other one is a substitued amino group, i.e., a compound of the following general formula (V), 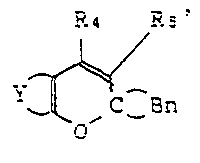 wherein 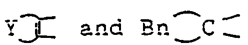 are the same as those of the above-mentioned general formulas (I) and (II),
and either one of R₄ and R₅' is a hydrogen atom and
the other one is a group substituted amino group may be obtained by reacting the spiropyrone compound of formula (III) with the primary amine or the secondary amine of the formula

R-H

wherein R is a substituted amino group which is the condensing agent used for the reaction of the compound of the general formula (I) with the compound of the general formula (II) under the same conditions as those of the above reaction. In this case, the primary amine or the secondary amine should be used in an amount of usually from 0.1 to 10 moles per mole of the spiropyrone compound. This process is usually carried out by executing the process of reacting the compound of the general formula (I) with the compound of the general formula (II) of the present invention in the presence of the primary amine or the secondary amine and, further, continuing the reaction by adding the above amine to the reaction solution. The reaction for obtaining the compound of the general formula (V) should be carried out for 5 to 20 hours as counted from the start of the reaction of the compound of the general formula (I) with the compound of the general formula (II).

In method (a) as set out in the claims, a compound of the general formula (IV) in which R₄ and R₅ are both hydrogen atoms, i.e., a compound of the following general formula (VI) 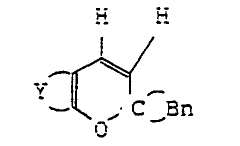 wherein 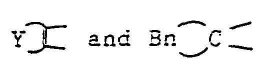 are the same as those of the above-mentioned general formulas (I) and (II), is obtained by, first, reducing the spiropyrone compound obtained by the present invention with a reducing agent to obtain a compound of formula (XI) 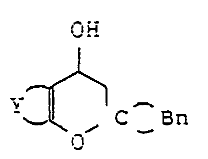 wherein 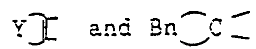 are the same as those of the above-mentioned general formulas (I) and (II), and, then, dehydrating this compound. Though there is no particular limitation, there is usually used, as the reducing agent, a hydrogen compound such as a sodium boron hydride or an aluminum lithium hydride. The reducing agent should be used in an amount of usually from 1 to 10 moles per a mole of the spiropyrone compound. The reaction temperature of the reducing reaction should be usually from 50 to 100°C and the reaction time is usually selected to be from 1 to 10 hours. Though there is no particular limitation in the reaction solvent, it is desired to use a polar solvent such as a methanol, an ethanol, a tetrahydrofurane or a dimethylformamide. The dehydration may be carried out by any method. Usually, however, the dehydration is carried out by separating the product after the reduction reaction has been finished and then adding a dehydrating agent thereto. Here, though there is no particular limitation, there is usually used, as the dehydrating agent, anhydrous copper sulfate, hydrochloric acid, magnesium sulfate or alumina. The dehydrating agent should be used in an amount of usually from 0.1 to 10 parts by weight per a part by weight of the spiropyrone compound. The reaction temperature of the dehydration reaction should usually be from 50 to 200°C, and the reaction time is usually selected to be from 0.1 to 10 hours.

In method (b) as set out in the claims, a compound of the general formula (IV) in which R₄ is an alkyl group, an aralkyl group or an aryl group, and R₅ is a hydrogen atom, i.e., a compound of the following general formula (VII) 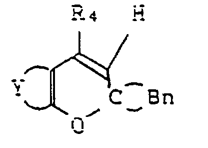 wherein 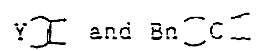 are the same as those of the above-mentioned general formulas (I) and (II), and R₄ is an alkyl group, an aralkyl group or an aryl group,
is obtained by, first, obtaining a compound of formula (XII) 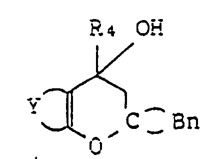 wherein 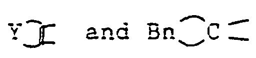 are the same as those of the above-mentioned general formulas (I) and (II), and R₄ is an alkyl group, an aralkyl group or an aryl group,
by reacting the spiropyrone compound obtained by the present invention with a compound of the formula

R-MgX

or

R-Li

wherein R is an alkyl group, an aryl group or an aralkyl group, and X is a halogen atom, and then dehydrating this compound. Here, as halogen for R-MgX, there should preferably be used bromine, chlorine or iodine. The organometal compound should be used usually in an amount of from 1 to 10 moles per a mole of the spiropyrone compound. The reaction temperature is usually from -20 to 70°C, and the reaction time is usually from 0.1 to 10 hours. As the solvent of the reaction, it is desired to use ethers such as an ethyl ether, a tetrahydrofurane or a dioxane. The compound obtained by the above reaction should be dehydrated by the same method as the one for obtaining the compound of the above-mentioned general formula (VI).

In method (d) as set out in the claims, a compound represented by the general formula (IV) wherein R₄ is a hydrogen atom and R₅ is an alkyl group, an aralkyl group or an aryl group, e.g., a compound of the following general formula (VIII) 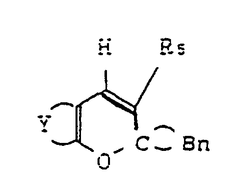 wherein 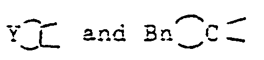 are the same as those of the above-mentioned general formulas (I) and (II), and R₅ is an alkyl group, an aralkyl group or an aryl group,
is obtained by, first, carrying out the process for preparing the spiropyran compound represented by the above-mentioned formula (IV), preferably formula (V) reacting the spiropyran compounds of the general formula (V) with a compound of the formula

R₅X

wherein R₅ is as defined above, to obtain a compound represented by the following general formula (IX) 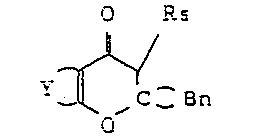 wherein 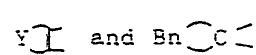 are the same as those of the above-mentioned general formulas (I) and (II), and R₅ is an alkyl group, an aralkyl group or an aryl group.
and reducing the thus obtained compound followed by dehydration. Here, iodine, bromine or chlorine is usually used as halogen for the compound of the formula R₅X. These halogen compounds should be used usually in an amount of 1 to 10 moles per a mole of the spiropyrone compound. The reaction temperature for obtaining the compound represented by the general formula (IX) should desirably be from -20 to 50° C, and the reaction time is usually from 0.1 to 10 hours. Though there is no particular limitation, desired examples of the solvent of the reaction are polar solvents such as a methanol, an ethanol, a toluene and a tetrahydrofurane. The compound represented by the general formula (IX) is reduced with a reducing agent and is then dehydrated in the same manner as the one carried out for obtaining the compound represented by the above-mentioned general formula (VI).

In method (e) as set out in the claims, a compound represented by the general formula (IV) wherein R₄ and R₅ are the same or different alkyl groups, aralkyl groups or aryl groups, i.e., a compound represented by the following general formula (X) 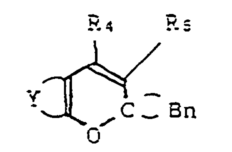 wherein 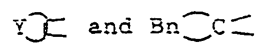 are the same as those of the above-mentioned general formulas (I) and (II), and R₄ and R₅ are the same or different alkyl groups, aralkyl groups or aryl groups,
is obtained by, first, obtaining a compound of formula 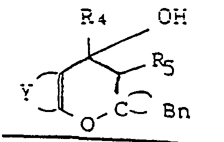 wherein 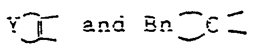 are the same as those of the above-mentioned general formulas (I) and (II), and R₄ and R₅ are the same or different alkyl groups, aralkyl groups or aryl groups,
by reacting the compound represented by the formula (IX) with a compound of formula R₄-MgX or R₄-Li wherein R₄ is as defined above and then dehydrating this compound.

The spiropyran compounds represented by the above general formula (IV) obtained by the aforementioned process usually exist in the form of colorless or pale yellow solids or viscous liquids at normal temperature and under normal pressure, and can be confirmed by the following means (a) to (c).
(a) The kind and number of protons existing in the molecules can be learned by the nuclear magnetic resonance spectrum of protons (H¹-NMR). That is, a peak appears near δ7 to 8.5 ppm due to an aromatic proton, broad peaks appear near δ1.2 to 3.5 ppm and near 5.3 to 5.7 ppm due to protons of a 2-bicyclo[3,3,1]9-nonenylidene group, and a peak appears near δ5.5 to 7.0 ppm due to protons of an alkene when R₄ and R₅ are hydrogen atoms. Further, the number of protons in the bonding group can be learned by comparing their δ peak intensities.
(b) Amounts in percent by weight of carbon, hydrogen, nitrogen, sulfur and halogen can be found by the elemental analysis. The amount in percent by weight of oxygen can be calculated by subtracting the sum of amounts in percent by weight of the above-found elements from 100. Therefore, the composition of the formed product can be determined.
(c) The kind of carbon present in the molecules can be learned by the ¹³C-nuclear magnetic resonance spectrum (¹³C-NMR). A peak appears near δ27 to 52 ppm due to carbon of the 2-bicyclo[3,3,1]9-nonenylidene group, a peak appears near δ15 to 35 ppm due to carbon of the alkyl group when R₄ and R₅ are alkyl groups, and a peak appears near δ110 to 150 ppm due to carbon of an aromatic hydrocarbon group or of an unsaturated heterocyclic group.

The spiropyran compound represented by the general formula (IV) dissolves well in a general organic solvent such as a toluene, a chloroform or a tetrahydrofuran. A spiropyran compound represented by the general formula (IV) that is dissolved in such a solvent is, usually, almost colorless but develops a color or quickly turns into a densely colored compound upon irradiation with the sunlight or ultraviolet rays, and quickly returns back to the initial colorless state when the light is interrupted, thus exhibiting a good reversible photochromic action. The photochromic action of the compound of the general formula (IV) takes place even in a high molecular solid matrix, the reversing speed being in the order of seconds. Any high molecular matrix can be used provided it permits a spiropyran compound represented by the general formula (IV) to be uniformly dispersed therein. Optically preferred examples thereof include polymers such as a methyl polyacrylate, an ethyl polyacrylate, a methyl polymethacrylate, an ethyl polymethacrylate, a polystyrene, a polyacrylonitrile, a polyvinyl alcohol, a polyacrylamide, a poly(2-hydroxyethylmethacrylate), a polydimethylsiloxane, a polycarbonate, a poly(allyldiglycol carbonate), or polymers obtained by copolymerizing monomers that form these polymers or by copolymerizing these monomers with other monomers.

As described above, the spiropyran compounds prepared from the spiropyrone compounds which are the starting materials obtained by the process of the present invention can be extensively used as a photochromic material. For example, they can be used as a variety of memory materials to substitute for silver salt photosensitive materials, and as copying materials, photosensitive materials for printing, recording materials for use with a cathode-ray tube, photosensitive materials for use with a laser beam, and photosensitive materials for holography. Furthermore, the photochromic materials using the spiropyran compounds can be utilized as such materials as a photochromic lens material, an optical filter material, a display material, an actinometer and ornamental materials. When used for the photochromic lens, for instance, there is no particular limitation on the method preparation provided a uniform dimming property is obtained. Concrete examples include a method of sandwiching in the lens a polymer film in which is uniformly dispersed the photochromic material of the present invention and a method in which the compound is dissolved in, for example, a silicone oil at a temperature of 150 to 200°C for 10 to 60 minutes so as to infiltrate into the lens surface which is then coated with a hardenable substance to obtain a photochromic lens. There can be further contrived a method in which the lens surface is coated with the polymer film which is then coated with a hardenable substance to obtain a photochromic lens.

When such a photochromic lens is to be obtained, it is desired to use a photochromic material that develops a dense color near the normal temperature upon irradiation with the sunlight. A compound suited for such a photochromic lens is the one represented by the above general formula (IV) wherein 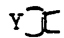 is a divalent group derived from a naphthalene ring, a phenanthrene ring, a pyridine ring and a quinoline ring. In particular, a compound in which both R4 and R5 are hydrogen atoms has such a merit that the color develops densely and fades quickly.

By selecting the substitutent R₄ or R₅ of the general formula (IV), furthermore, it is allowed to change the color-fading speed of the compound of the general formula (IV). For instance, when R₄ and R₅ are alkyl groups, a high color-fading speed is obtained presumably because the trans form is less established in the color-developing state. When R4 is a substituted amino group, the trans form in the color- developing state is stabilized by the resonance, and a dense color develops accompanied, however, by a slight decrease in the color-fading speed. The substituents R₄ and R₅ can be arbitrarily selected depending upon the object.

In the spiropyrone compounds obtained by the process of the present invention, the group bonded to the second position of the benzopyran ring or the naphthopyran ring is a 2-bicyclo[3,3,1]9-nonelidene group. As a result, the spiropyrone compounds can be obtained in very good yields.

The spiropyran compounds represented by the general formula (IV) prepared from the spiropyrone compounds which are the starting materials develop yellow or orange color from their colorless state at normal temperature (10 to 40°C) or at temperatures slightly higher than the normal temperature upon irradiation with ultraviolet rays, return from the color-developing state back to the colorless state within short periods of time, and develop a dense color, thus exhibiting excellent photochromic properties.
Therefore, the present invention is very utilizable as a process for preparing in good yields the spiropyrone compounds which are the starting materials from which the spiropyran compounds can be efficiently prepared.

### Examples

The present invention will be described below in further detail by way of Examples, but it should be noted chat the invention is in no way limited to these Examples only.

### Example 1

A solution was prepared by dissolving 11.16 g (0.06 mol) of a 1-hydroxy-2-acetonaphthone and 8.16 g (0.06 mol) of a 2-bicyclo[3,3,1]nonene-9-one in 300 cc of toluene. The mixture was boiled for 10 hours, and water was separated by the azeotropic dehydration. After the reaction, toluene was removed under reduced pressure, and the remaining product was crystallized with acetone to obtain a spiropyrone compound of the following formula in an amount of 15.6 g (yield, 85%).

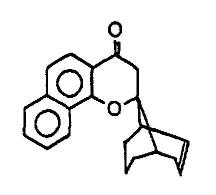

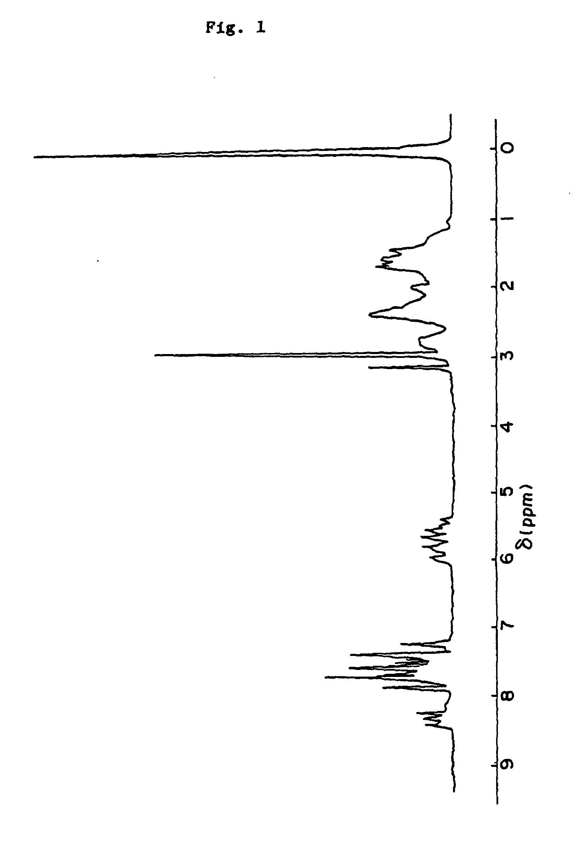

Elemental analysis of this compound indicated C 82.89%, H 6.62%, 0 10.49% which were in very good agreement with the calculated values of C₂₁H₂₀O₂, i.e., C 82.68%, H 6.61%, O 10.51%. Furthermore, the nuclear magnetic resonance spectra of protons (Fig. 1) indicated a peak of 4H near 7.2 to 8.3 ppm due to the protons of a naphthalene ring, a peak of 2H near 5.3 to 6.7 ppm due to the protons of an alkene, and broad peaks of 12H near 1.2 to 3.5 ppm due to the protons of a 2-bicyclo[3,3,1]9-nonenylidene group and protons at the third position of a spiropyrone ring. Furthermore, the ¹³C nuclear magnetic resonance spectra indicated a peak near 25 to 55 ppm due to carbon of the 2-bicyclo[3,3,1]9-nonenylidene group and carbon at the third position of the spiropyrone ring, a peak near 110 to 160 ppm due to carbon of the naphthalene ring, and a peak near 80 to 140 ppm due to carbon of the alkene. It was confirmed from the above results that the isolated product was a compound represented by the above structural formula.

### Example 2

A solution was prepared by dissolving 11.16 g (0.06 mol) of a 1-acetyl-2-naphthol, 8.16 g (0.06 mol) of a 2-bicyclo[3,3,1]nonene-9-one and 8.7 g (0.1 mol) of a morpholine in 300 cc of toluene. The mixture was boiled for 5 hours, and water was separated by the azeotripic dehydration. After the reaction, toluene was removed under reduced pressure, and the remaining product was crystallized with acetone to obtain a spiropyrone compound of the following formula in an amount of 14.6 g (yield, 80%).

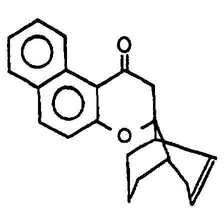

Elemental analysis of this compound indicated C 82.84%, H 6.63%, O 10.51% which were in very good agreement with the calculated values of C₂₁H₂₀O₂, i.e., C 82.68%, H 6.61%, O 10.51%. Furthermore, the nuclear magnetic resonance spectra of protons indicated a peak of 4H near 7.2 to 8.3 ppm due to the protons of a naphthalene ring, a peak of 2H near 5.3 to 6.7 ppm due to the protons of an alkene, and broad peaks of 12H near 1.2 to 3.5 ppm due to the protons of a 2-bicyclo[3,3,1]9-nonenylidene group and protons at the third position of a spiropyrone ring. Furthermore, the ¹³C nuclear magnetic resonance spectra indicated a peak near 25 to 55 ppm due to carbon of the 2-bicyclo[3,3,1]9-nonenylidene group and carbon at the third position of the spiropyrone ring, a peak near 110 to 160 ppm due to carbon of the naphthalene ring, and a peak near 80 to 140 ppm due to carbon of the alkene. It was confirmed from the above results that the isolated product was a compound represented by the above structural formula.

### Example 3

A reaction solution obtained through the same reaction as that of Example 2 and from which water was separated, was mixed with 6.1 g of a concentrated hydrochloric acid, and was washed with water and from which the organic layer was separated. Then, toluene was removed under reduced pressure, and the remaining product was recrystallized with acetone to obtain a spiropyrone compound of the above general formula in an amount of 15.6 g (yield 85%).

### Examples 4 to 26 and Comparative Examples 1 to 8

Various spiropyrone compounds were synthesized from the starting materials shown in Tables 1 and 2 in the same manner as in Example 1. The obtained spiropyrone compounds of Examples 4 to 26 were analyzed for their structures using the same means for confirming the structure as that of Example 1, and it was confirmed that the compounds were those represented by the structural formulas shown in Table 1. Table 3 shows values of elemental analysis of these compounds, values calculated from the structural formulas of the compounds and characteristic absorption in the nuclear magnetic resonance spectra of protons.

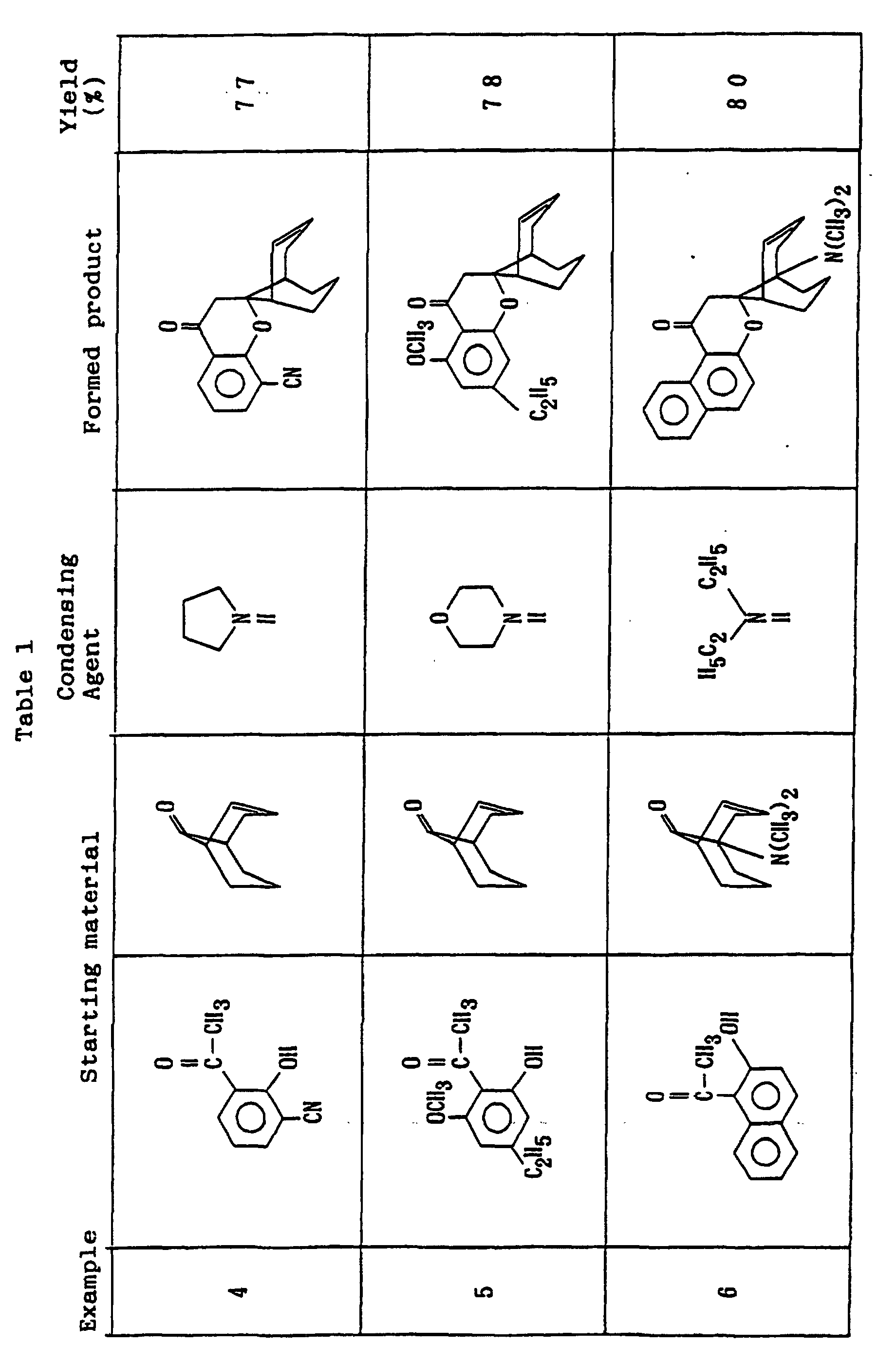

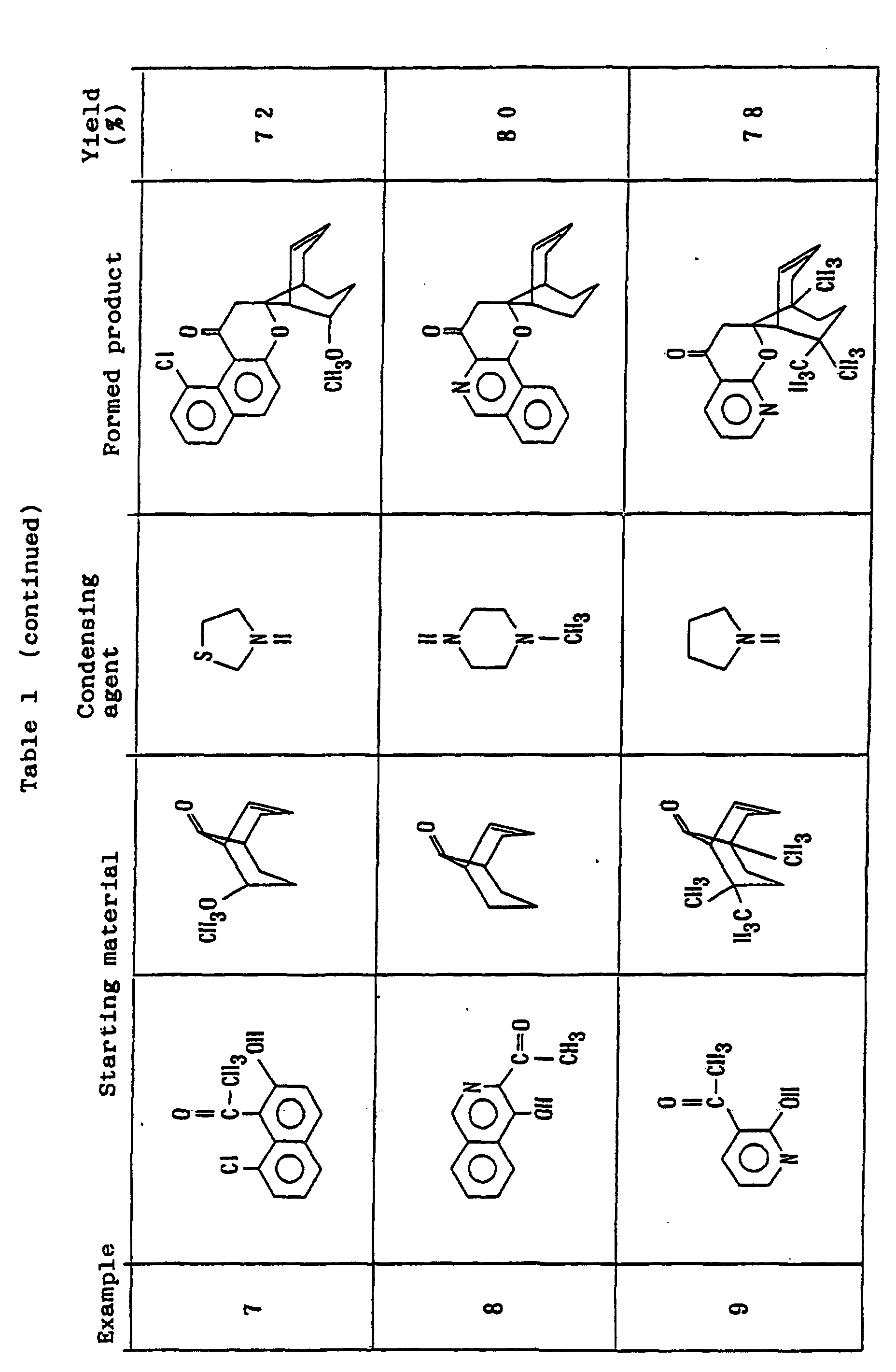

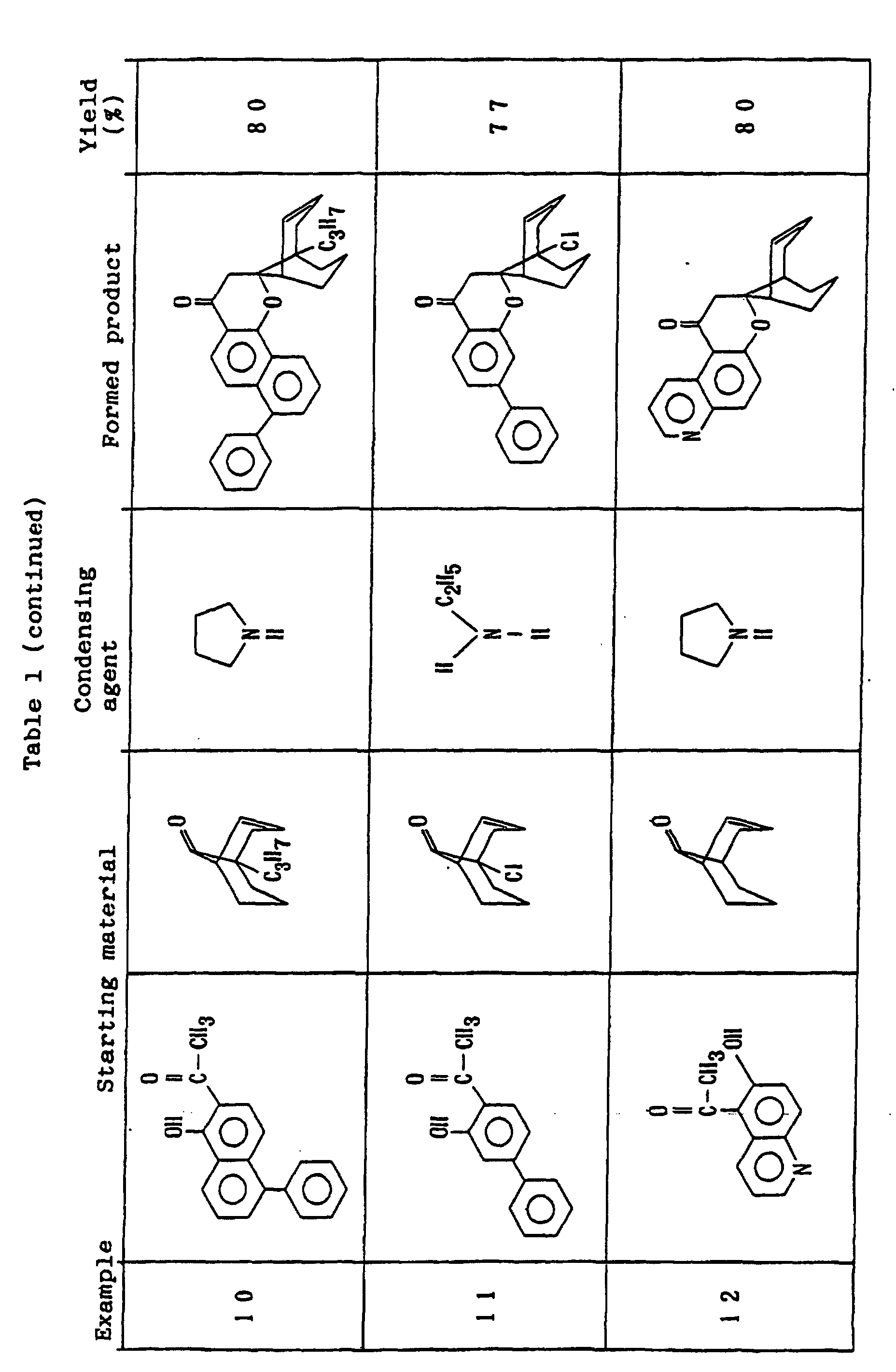

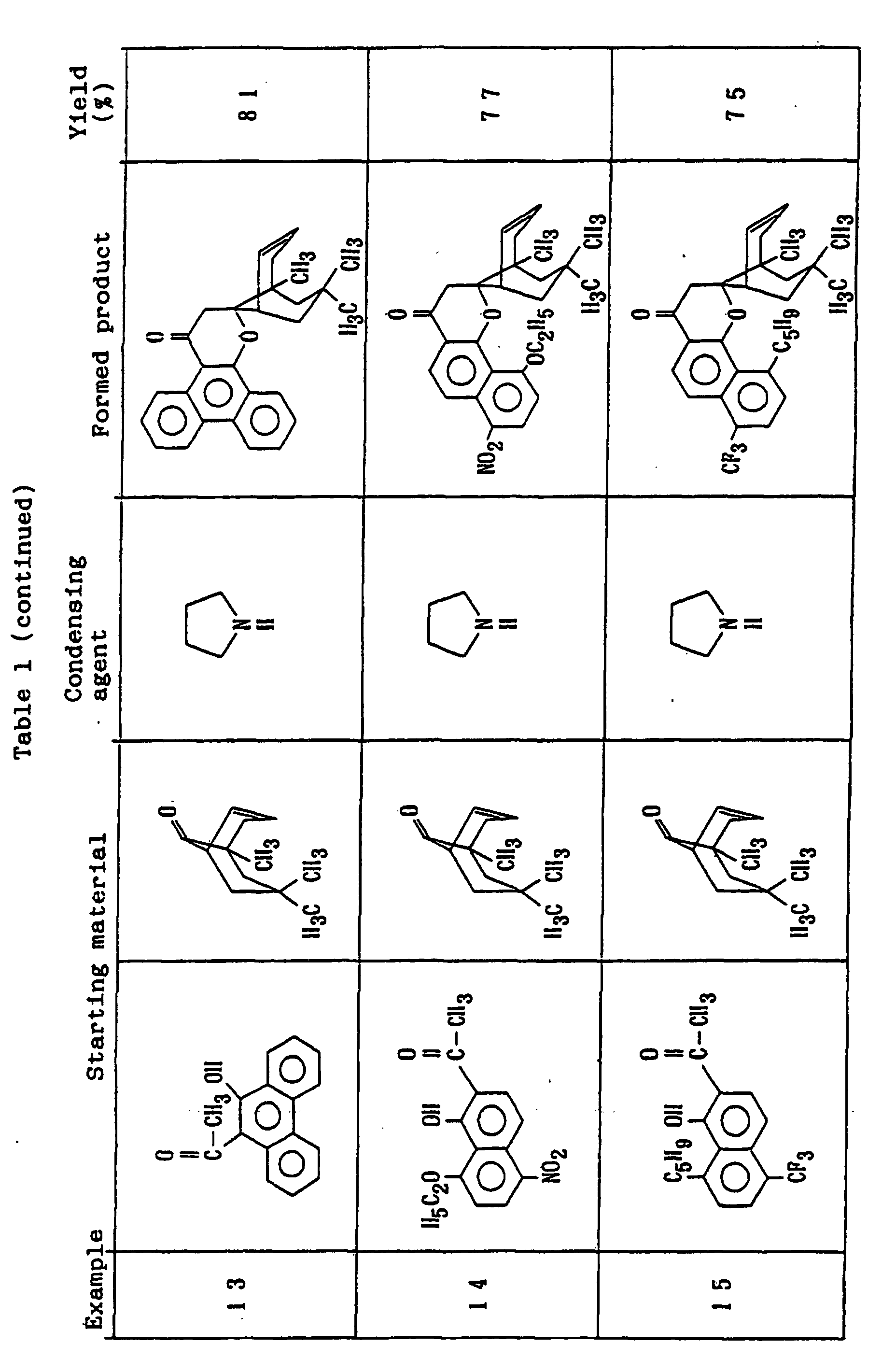

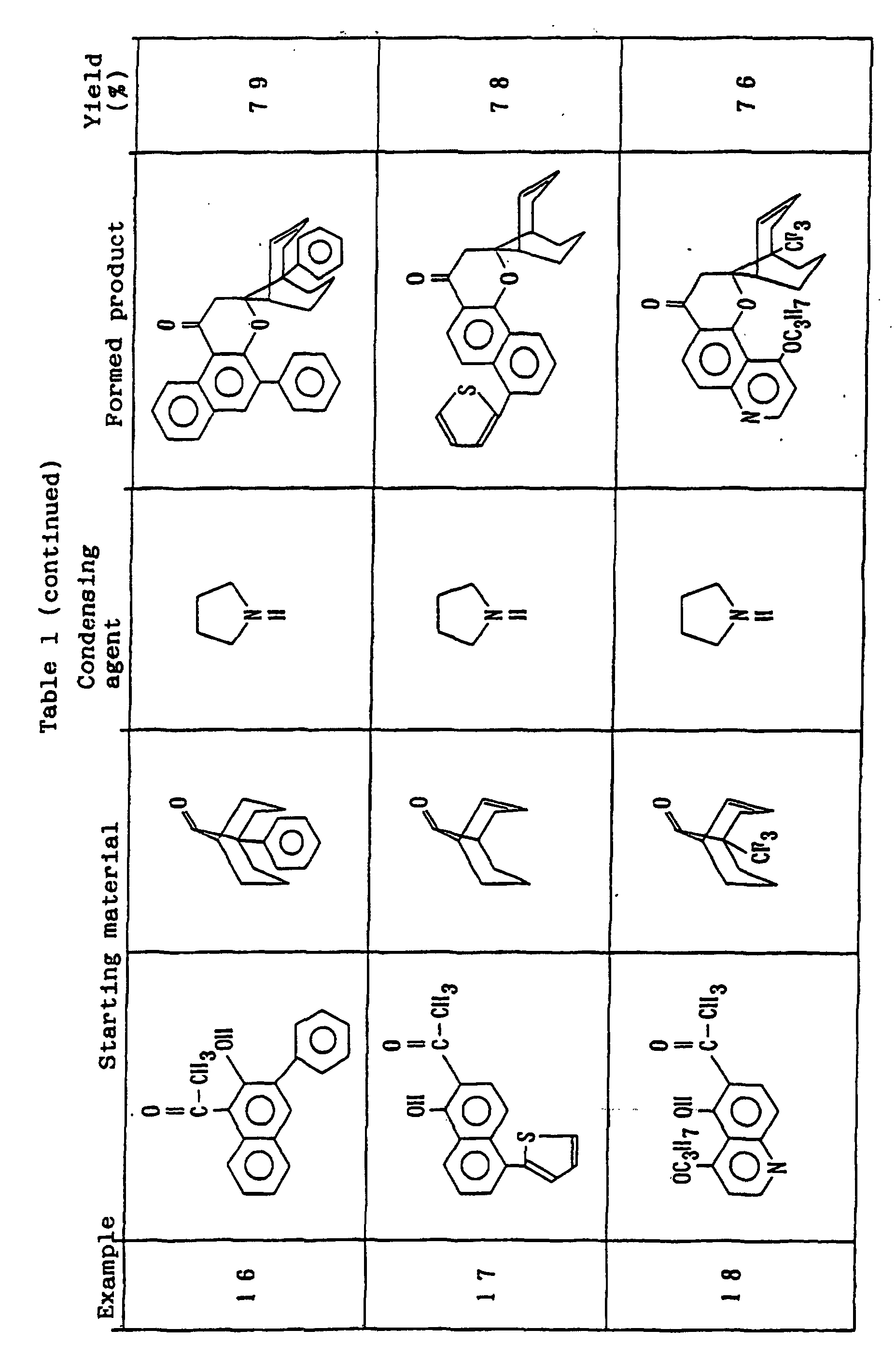

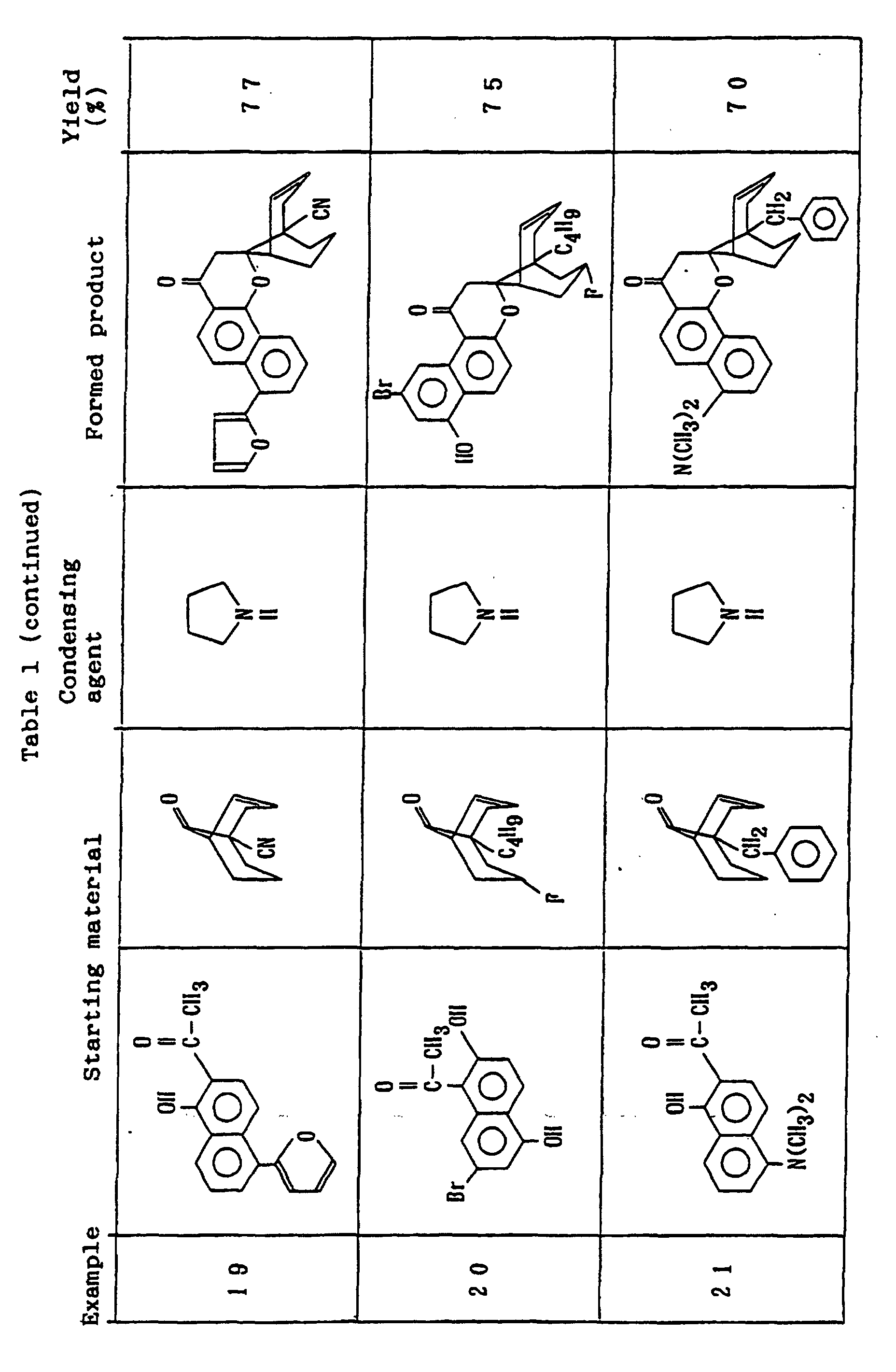

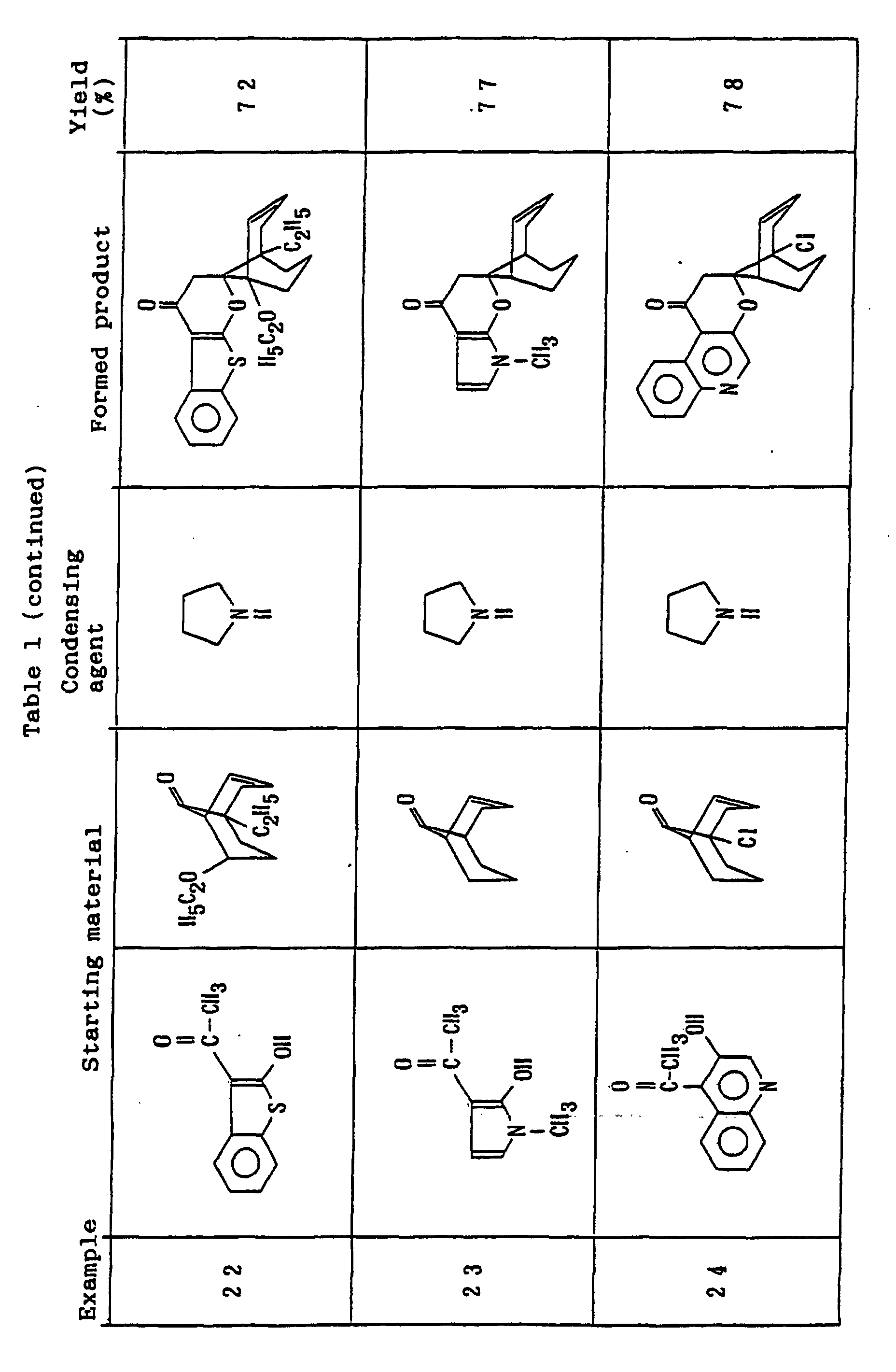

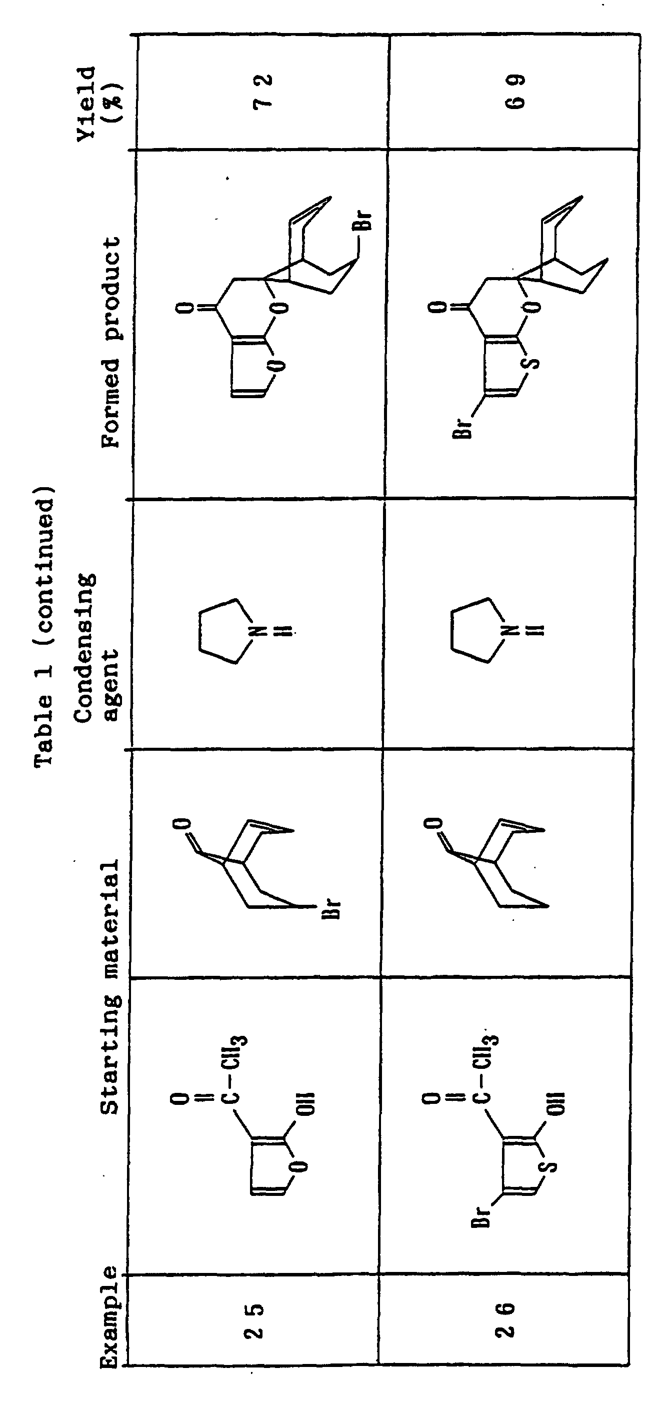

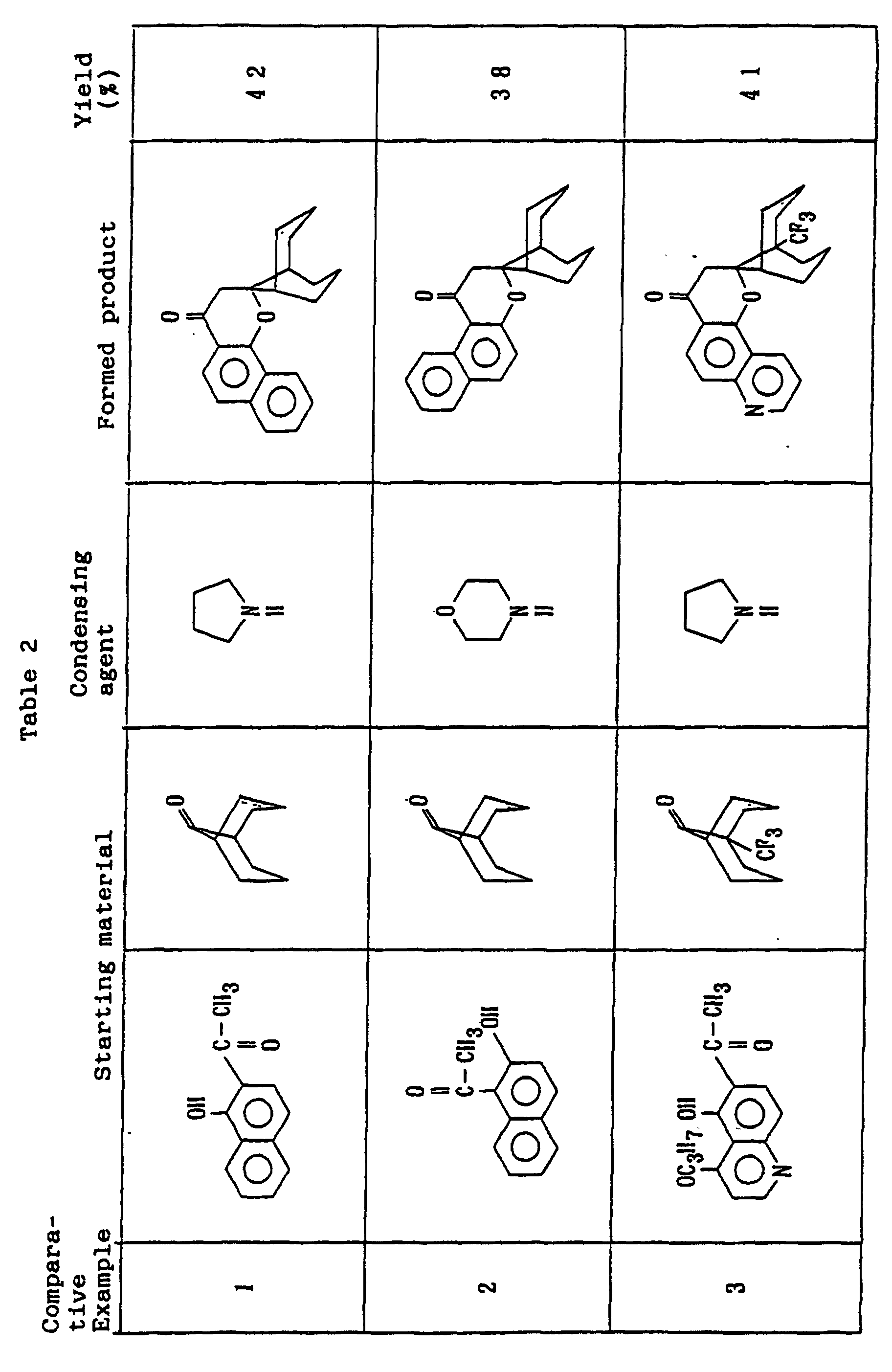

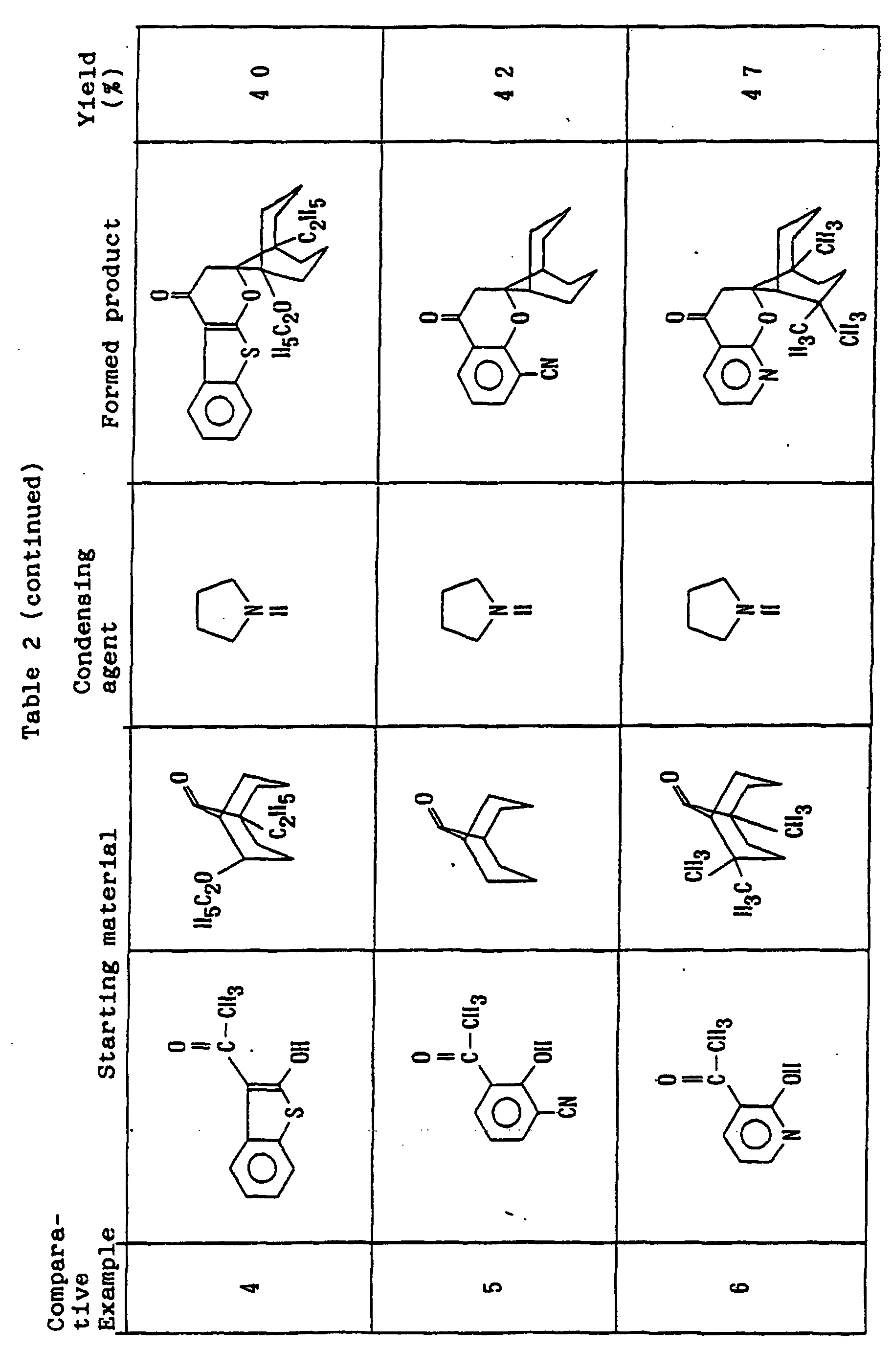

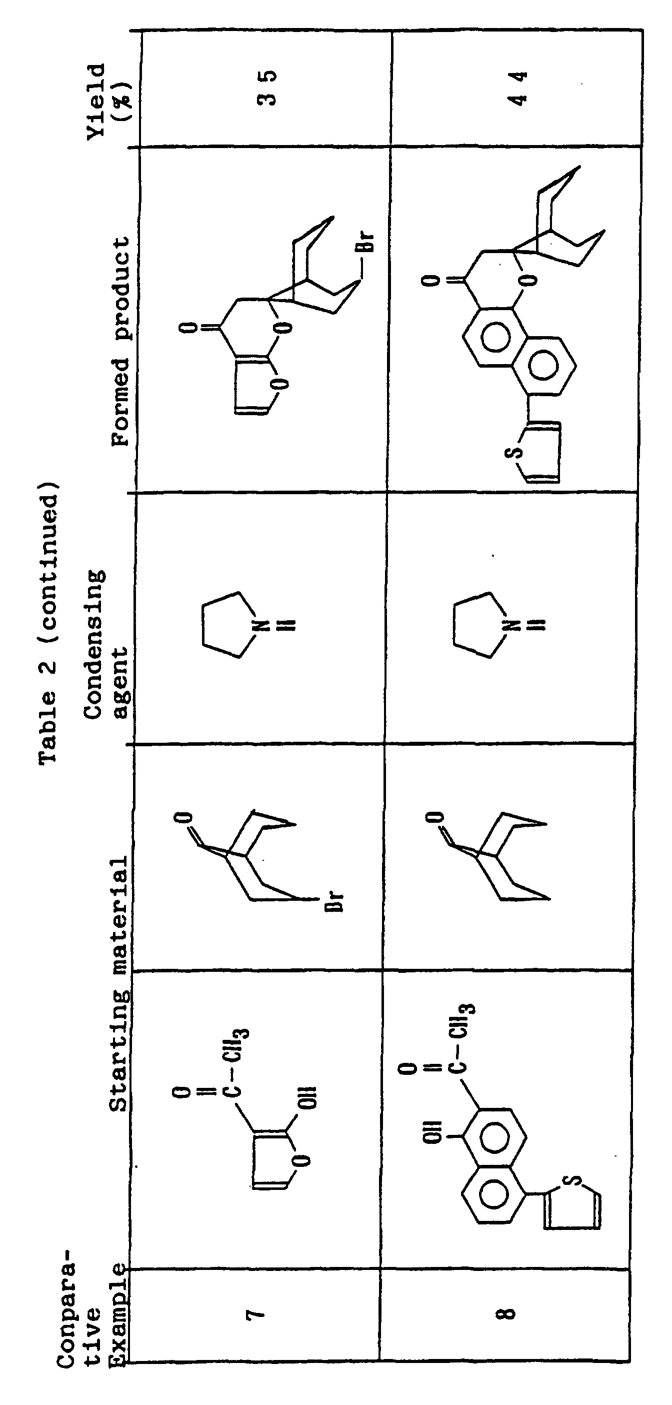

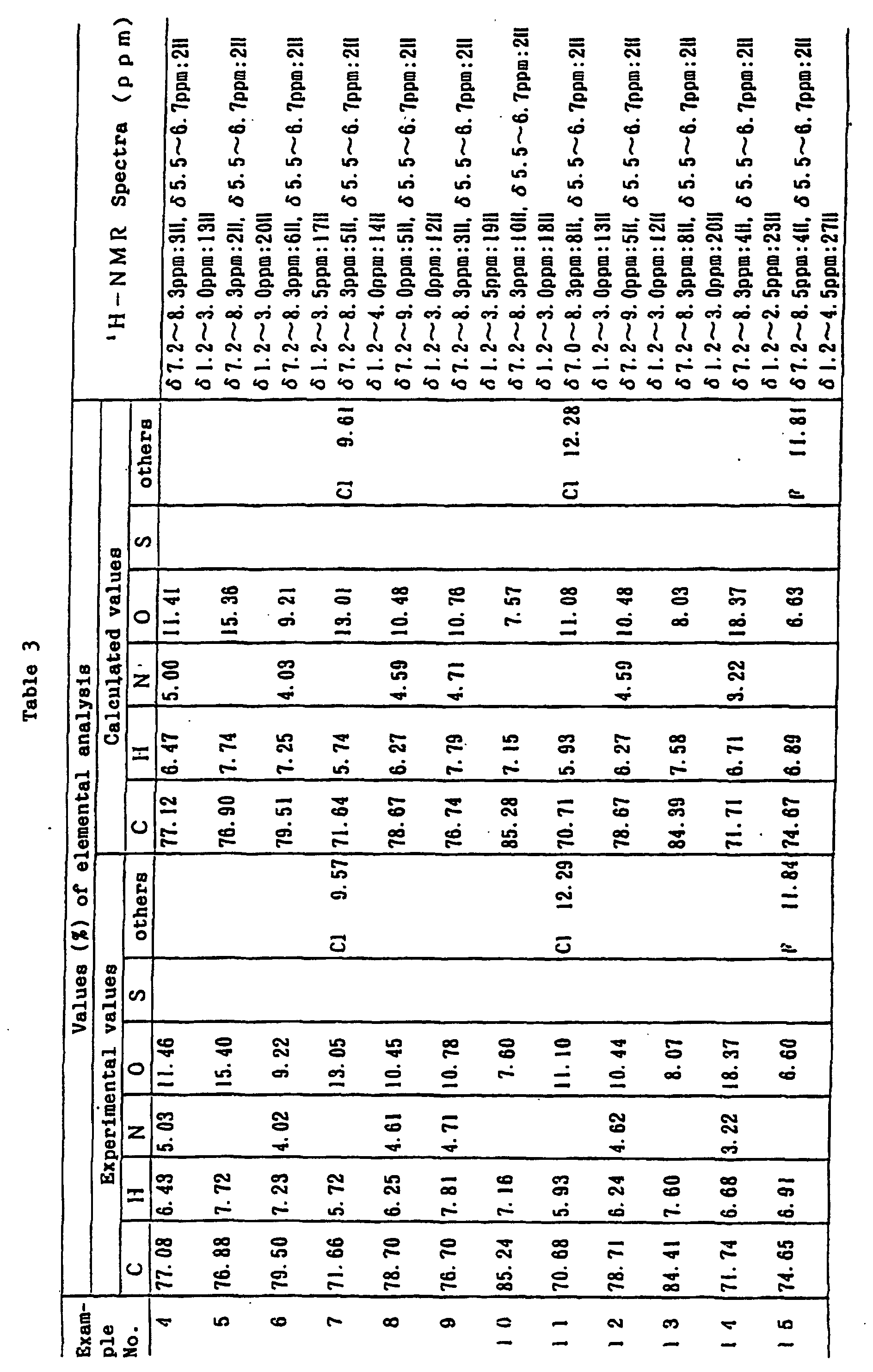

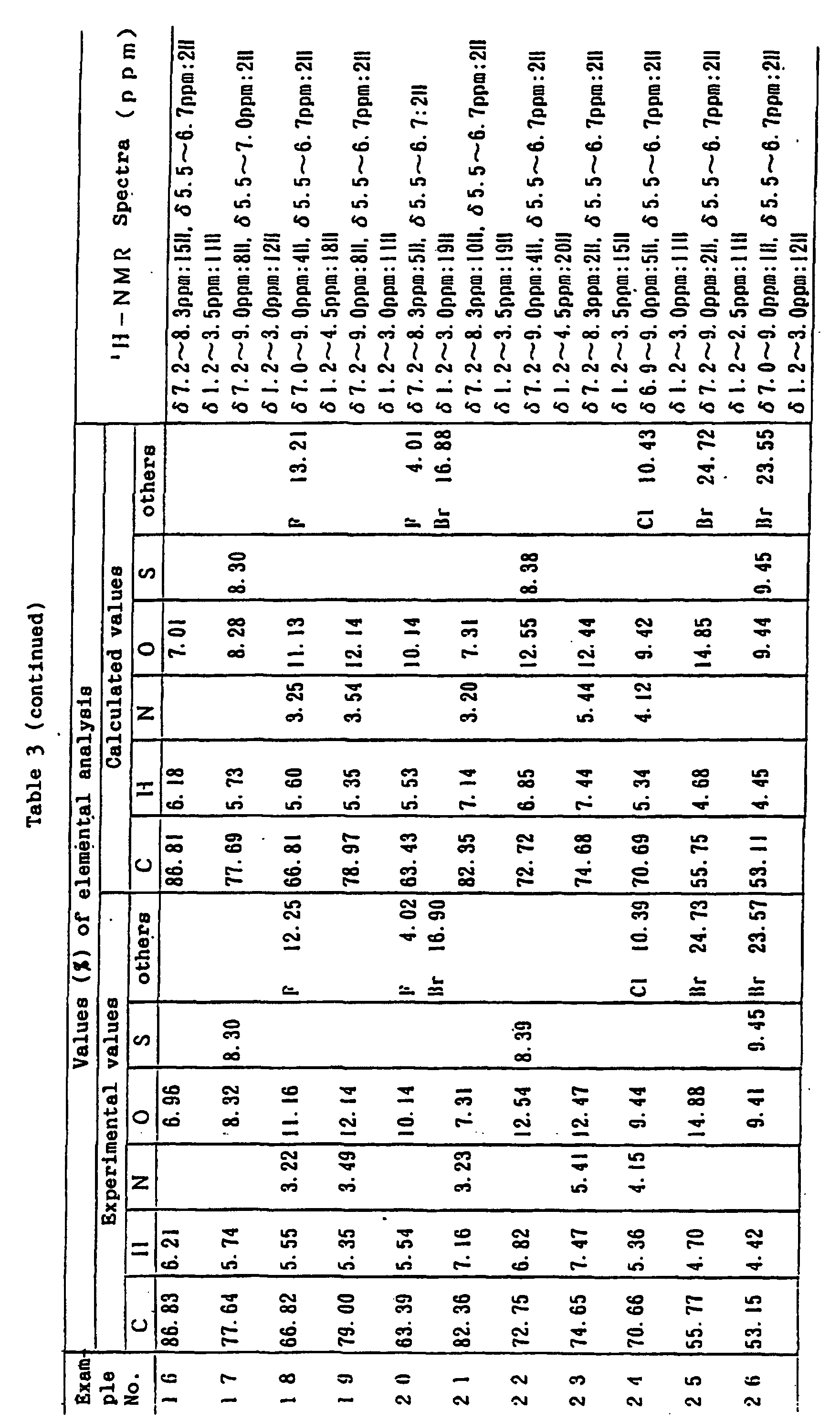

### Application Example 1

15.6 Grams (0.051 mol) of the spiropyrone compound of the following formula obtained in Example 1 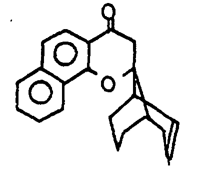 was dissolved in 200 cc of methanol, followed by the gradual addition of 3.42 g (0.09 mol) of a sodium boron hydride at a temperature of 65°C to carry out the reaction for 4 hours to obtain a 4-hydroxypiran compound. 17.4 Grams of the 4-hydroxypiran compound was heated together with 15 g of anhydrous copper sulfate in a stream of carbon dioxide at a temperature of 150 to 160°C for 10 minutes, and a brown viscous liquid was refined by chromatography on the silica gel to obtain a spiropyran compound of the following formula in an amount of 13.3 g. The yield of the spiropyran compound was 77% with respect to the amount of the 1-hydroxy-2-acetonaphtone that was used.

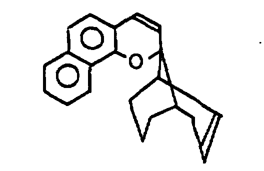

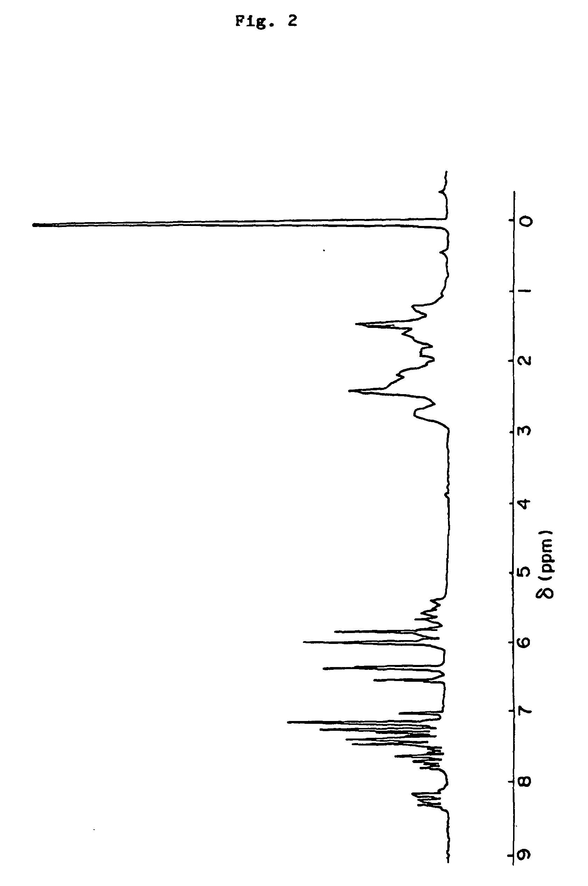

Elemental analysis of this compound indicated C 87.34%, H 7.04%, 0 5.62% which were in very good agreement with the calculated values of C₂₁H₂₀O, i.e., C 87.46%, H 6.99%, O 5.55%. Furthermore, the nuclear magnetic resonance spectra of protons (Fig. 2) indicated a peak of 6H near 7.2 to 8.3 ppm due to the protons of a naphthalene ring, a peak of 4H near 5.3 to 6.7 ppm due to the protons of an alkene, and a broad peak of 10H near 1.2 to 3.0 ppm due to the protons of a 2-bicyclo[3,3,1]9-nonenylidene group and protons at the third position of a spiropyrone ring. Furthermore, the ¹³C nuclear magnetic resonance spectra indicated a peak near 25 to 55 ppm due to carbon of the 2-bicyclo[3,3,1]9-nonenylidene group, a peak near 110 to 160 ppm due to carbon of the naphthalene ring, and a peak near 80 to 140 ppm due to carbon of the alkene. It was confirmed from the above results that the isolated product was a compound represented by the above structural formula.

### Application Example 2

14.6 Grams (0.048 mol) of the spiropyrone compound of the following formula obtained in Example 2 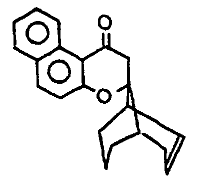 was dissolved in 200 cc of methanol, followed by the gradual addition of 2.74 g (0.072 mol) of an aluminum lithium hydride at a temperature of 65° C to carry out the reaction for 4 hours to obtain a 4-hydroxypiran compound of the following formula.

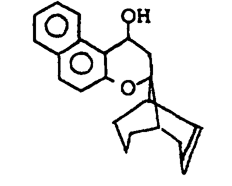

14.0 Grams of the 4-hydroxypiran compound was heated together with 15 g of anhydrous copper sulfate in a stream of carbon dioxide at a temperature of 170 to 180°C for 10 minutes, and a brown viscous liquid was refined by chromatography on the silica gel to obtain a spiropyran compound of the following formula in an amount of 12.6 g. The yield of the spiropyran compound was 72% with respect to the amount of the 1-acetyl-2-naphthol that was used.

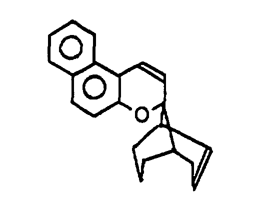

Elemental analysis of this compound indicated C 87.36%, H 7.02%, 0 5.62% which were in very good agreement with the calculated values of C₂₁H₂₀O, i.e., C 87.46%, H 6.99%, 0 5.55%. Furthermore, the nuclear magnetic resonance spectra of protons indicated a peak of 6H near 7.2 to 8.3 ppm due to the protons of a naphthalene ring, a peak of 4H near 5.5 to 7.0 ppm due to the protons of an alkene, and a broad peak of 10H near 1.2 to 3.0 ppm due to the protons of a 2-bicyclo[3,3,1]9-nonenylidene group. Furthermore, the 13C nuclear magnetic resonance spectra indicated a peak near 27 to 55 ppm due to carbon of the 2-bicyclo[3,3,1]9-nonenylidene group, a peak near 110 to 160 ppm due to carbon of the naphthalene ring, and a peak near 80 to 140 ppm due to carbon of the alkene. It was confirmed from the above results that the isolated product was a compound represented by the above structural formula.

### Application Example 3

3.04 Grams (0.01 mol) of a spiropyrone compound of the following formula obtained in the same manner as in Example 2 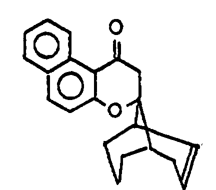 was dissolved in 50 cc of anhydrous ether. The solution was then cooled to 0°C, and a Grignard reagent CH₃MgI (0.012 mol) newly prepared in 50 cc of anhydrous ether was dropwisely added to the solution over a time of about one hour. After the dropwise addition has been finished, the solution was stirred at room temperature for another two hours. The ether solution was then calmly poured into cold water, the product was extracted with ether, the solution was dried with magnesium sulfate, the ether was removed under reduced pressure, and the spiropyrone compound was transformed into a 4-hydroxypyran compound of the following formula. 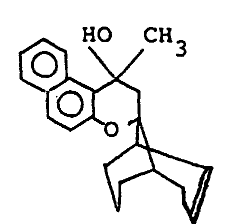 2.9 Grams of the 4-hydroxypyran compound was heated together with 3 g of anhydrous copper sulfate at 200°C for about 10 minutes in a stream of carbon dioxide, and a brown viscous solution was refined by chromatography on silica gel to obtain 2.57 g of a spiropyran compound of the following formula. The yield of the spiropyran compound was 68% with respect to the amount of the 1-acetyl-2-naphthol that was used.

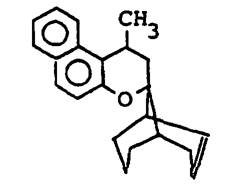

Through the elemental analysis, magnetic resonance spectra of protons and ¹³C-nuclear magnetic resonance spectra carried out in the same manner as in Example 1, it was confirmed that the compound was the one represented by the above structural formula. Table 5 shows values of elemental analysis of this compound and values calculated from the composition formula of this compound.

### Application Example 4

30.4 Grams (0.1 mol) of a spiropyrone compound of the following formula 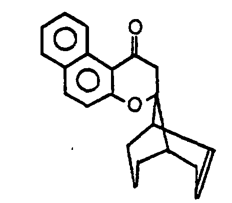 was prepared in the same manner as in Example 2, and to the reaction solution was further added 8.7 g (0.1 mol) of morpholine to continue the reaction for 10 hours. After the reaction, the toluene was removed under reduced pressure, and the remaining product was recrystallized with acetone to obtain 35.3 g of a compound represented by the following formula. The yield of the spiropyran compound was 82% with respect to the amount of the 1-acetyl-2-naphthol that was used.

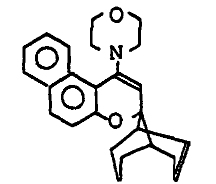

Through the elemental analysis, magnetic resonance spectra of protons and ¹³C-nuclear magnetic resonance spectra carried out in the same manner as in Example 1, it was confirmed that the compound was the one represented by the above structural formula. Table 5 shows values of elemental analysis of this compound and values calculated from the composition formula of this compound.

### Application Example 5

17.9 Grams (0.052 mol) of the spiropyran compound obtained in Application Example 4 was dissolved in 100 cc of methanol and was reacted with 8.12 g of a methyl iodide (0.057 mol) at a temperature of 10°C for 5 hours to obtain 15.6 g of a spiropyrone compound represented by the following formula.

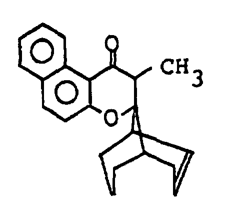

The thus formed spiropyrone compound was transformed into a 4-hydroxypyran compound in the same manner as in Application Example 1, subjected to the dehydration reaction, separated and was refined to obtain 13.3 g of a spiropyran compound of the following formula. The yield of the spiropyran compound was 68% with respect to the amount of the 1-acetyl-2-naphthol that was used.

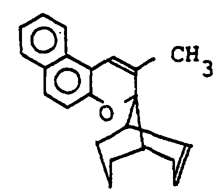

Through the elemental analysis, magnetic resonance spectra of protons and ¹³C-nuclear magnetic resonance spectra carried out in the same manner as in Example 1, it was confirmed that the compound was the one represented by the above structural formula. Table 5 shows values of elemental analysis of this compound and values calculated from the composition formula of this compound.

### Application Example 6

15.6 Grams (0.049 mol) of a spiropyrone compound of the following formula obtained in the same manner as in Application Example 5 was reacted with CH₃MgI in the same manner as in Application Example 3 to obtain 4-hydroxypiran compound 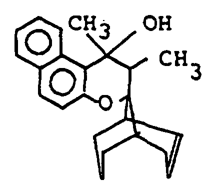 and was then subjected to the dehydration reaction to obtain 13.2 g of a spiropyran compound of the following formula. The yield of the spiropyran compound was 68% with respect to the amount of the 1-acetyl-2-naphthol that was used.

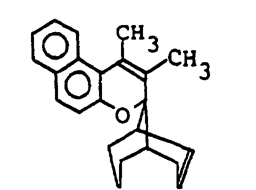

Through the elemental analysis, magnetic resonance spectra of protons and ¹³C-nuclear magnetic resonance spectra carried out in the same manner as in Example 1, it was confirmed that the compound was the one represented by the above structural formula. Table 5 shows values of elemental analysis of this compound and values calculated from the composition formula of this compound.

### Application Examples 7 to 42

Various spiropyran compounds were synthesized in the same manner as in Application Examples 1 to 6 from the spiropyrone compounds shown in Table 4 obtained in Examples 4 to 26. In Table 4, however, Application Examples 7 to 11 were carried out in the same manner as in Application Example 4, Application Examples 12 to 27 were carried out in the same manner as in Application Example 1 or 2, Application Examples 28 to 32 were carried out in the same manner as in Application Example 3, Application Examples 33 to 37 were carried out in the same manner as in Application Example 5, and Application Examples 38 to 42 were carried out in the same manner as in Application Example 6.

Analysis of the structures using the same means for confirming the structures as that of Example 1 indicated that the formed products were the compounds represented by the structural formulas shown in Table 4. Table 5 shows values of elemental analysis of the compounds, values calculated from the structural formulas of the compounds and characteristic absorption in the infrared-ray absorption spectrum.

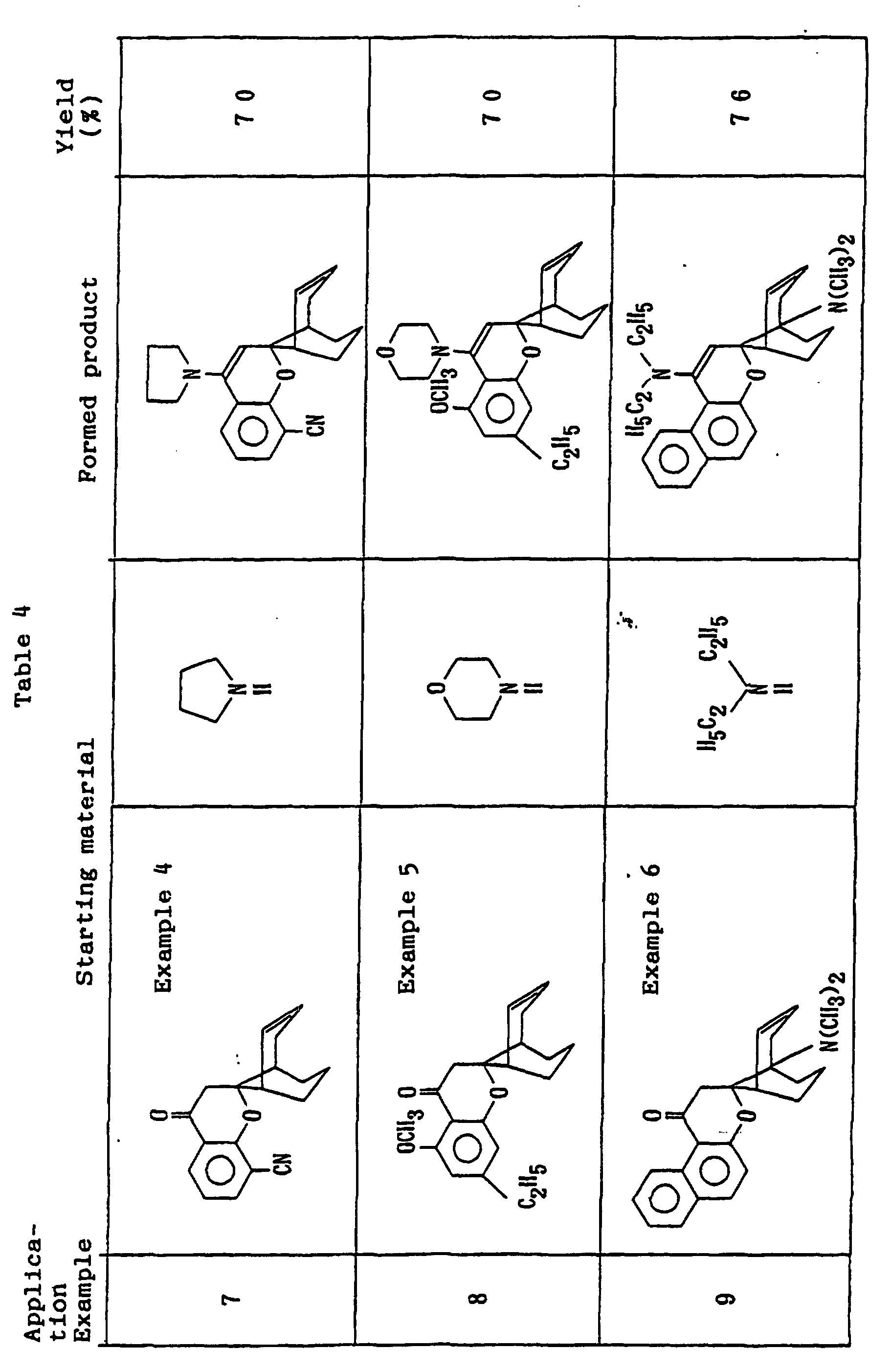

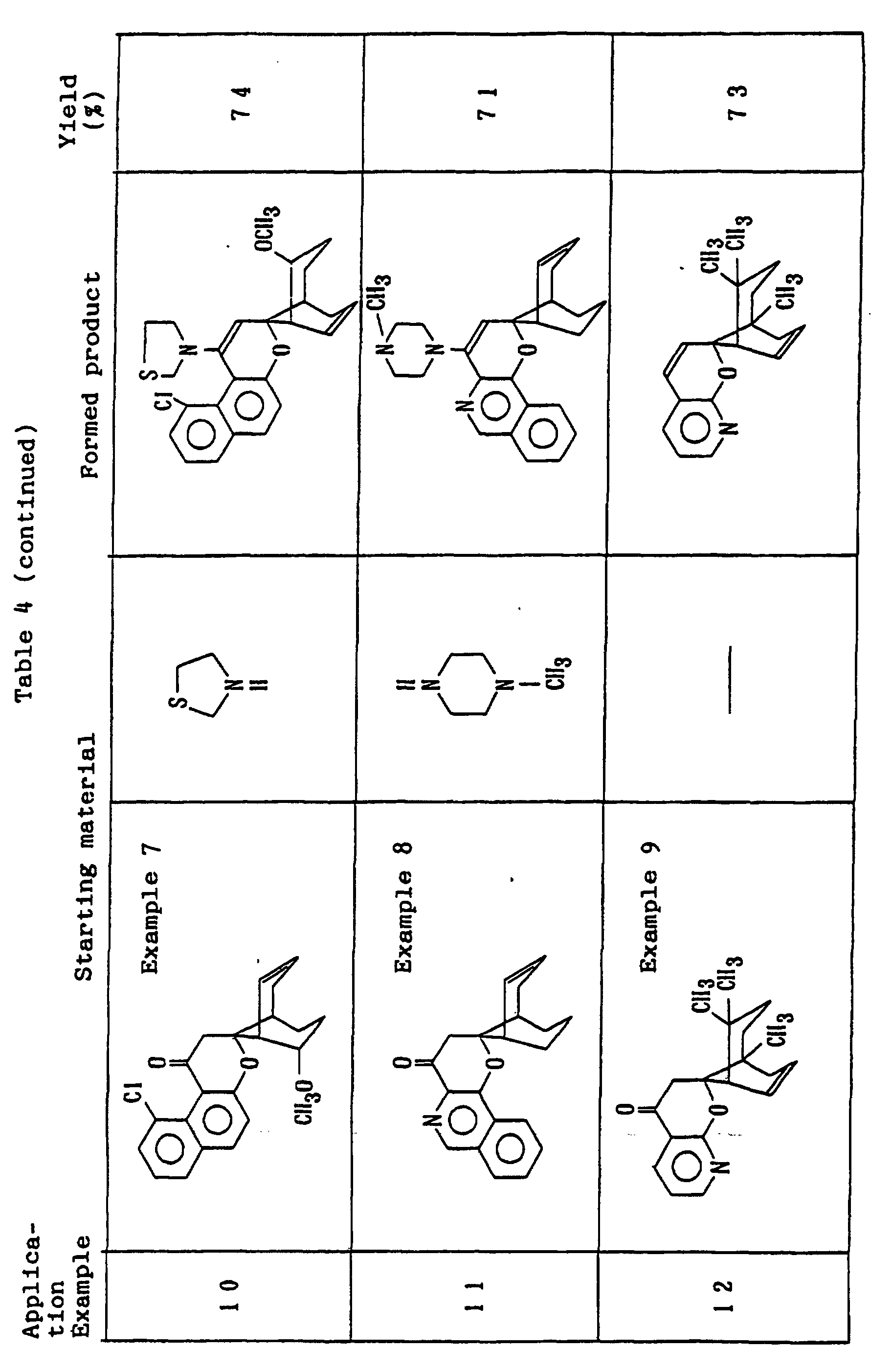

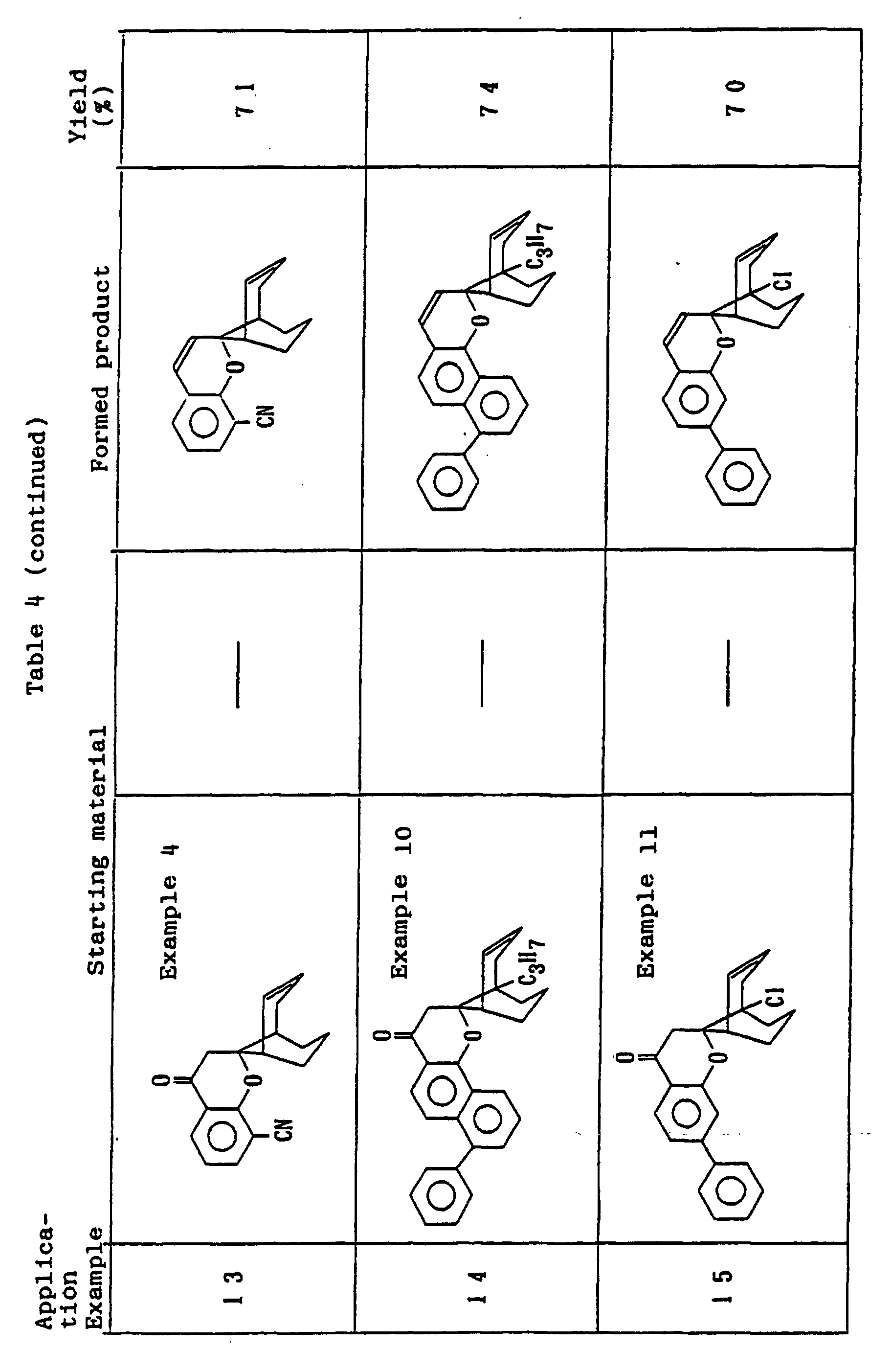

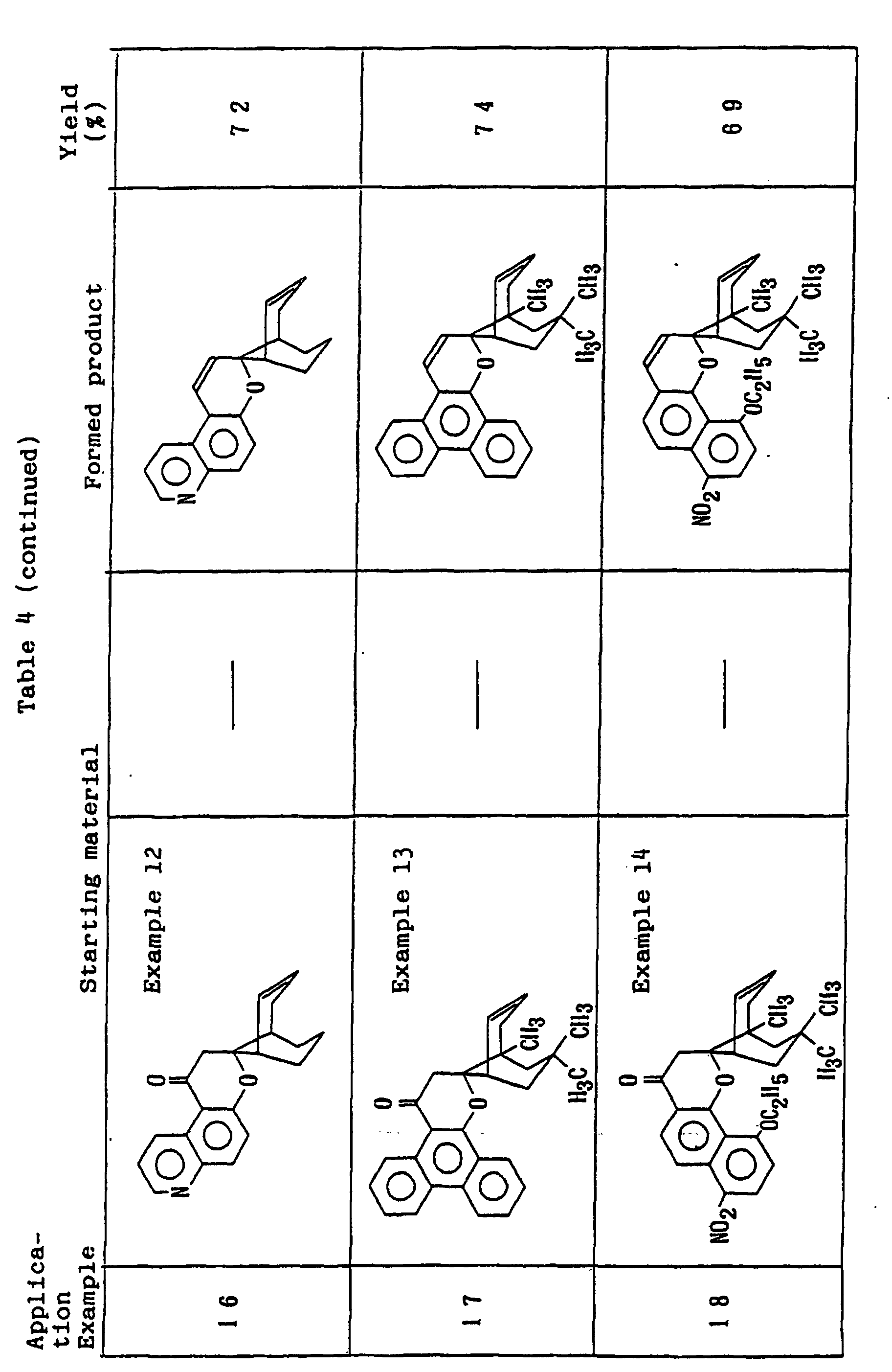

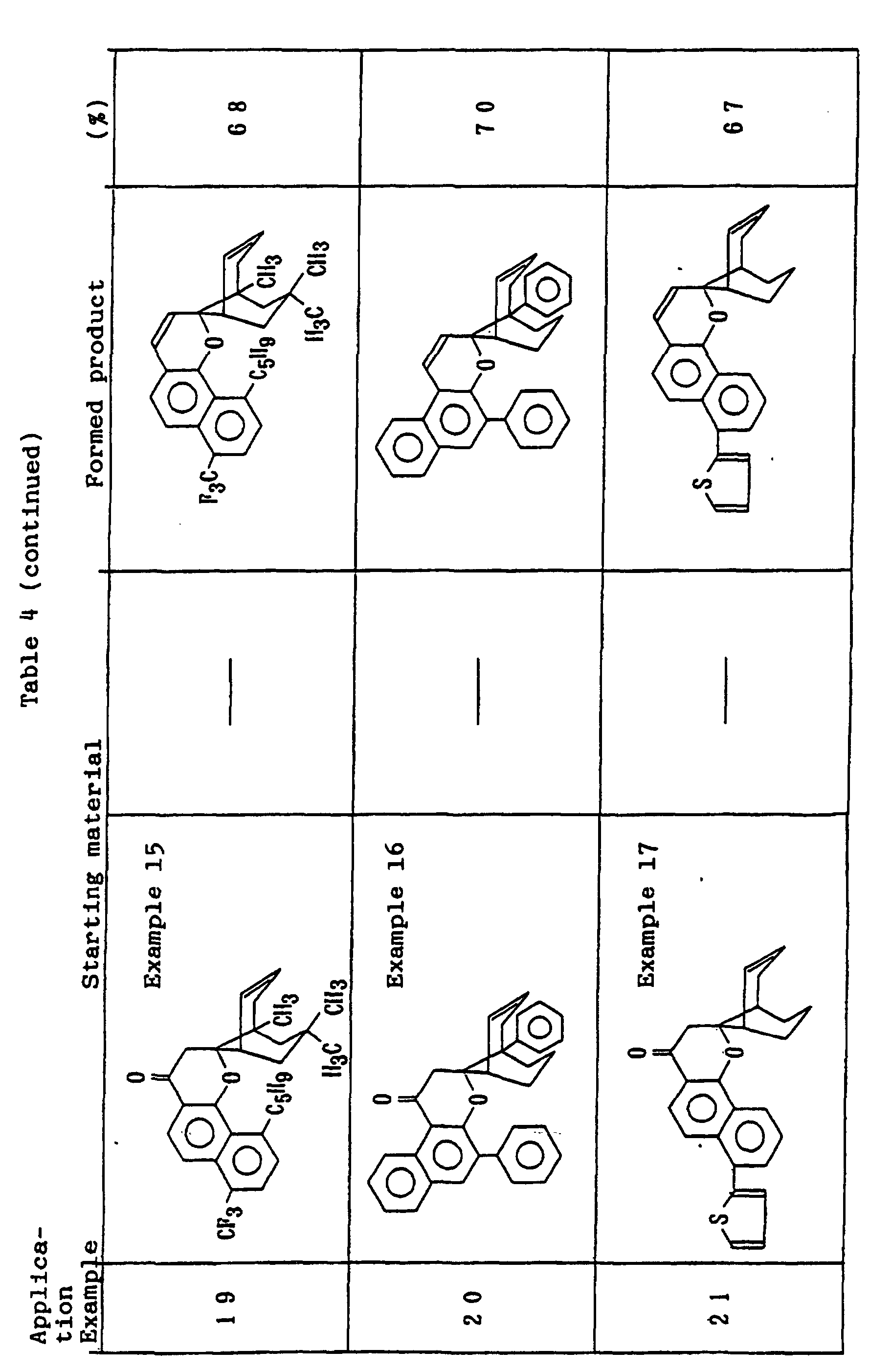

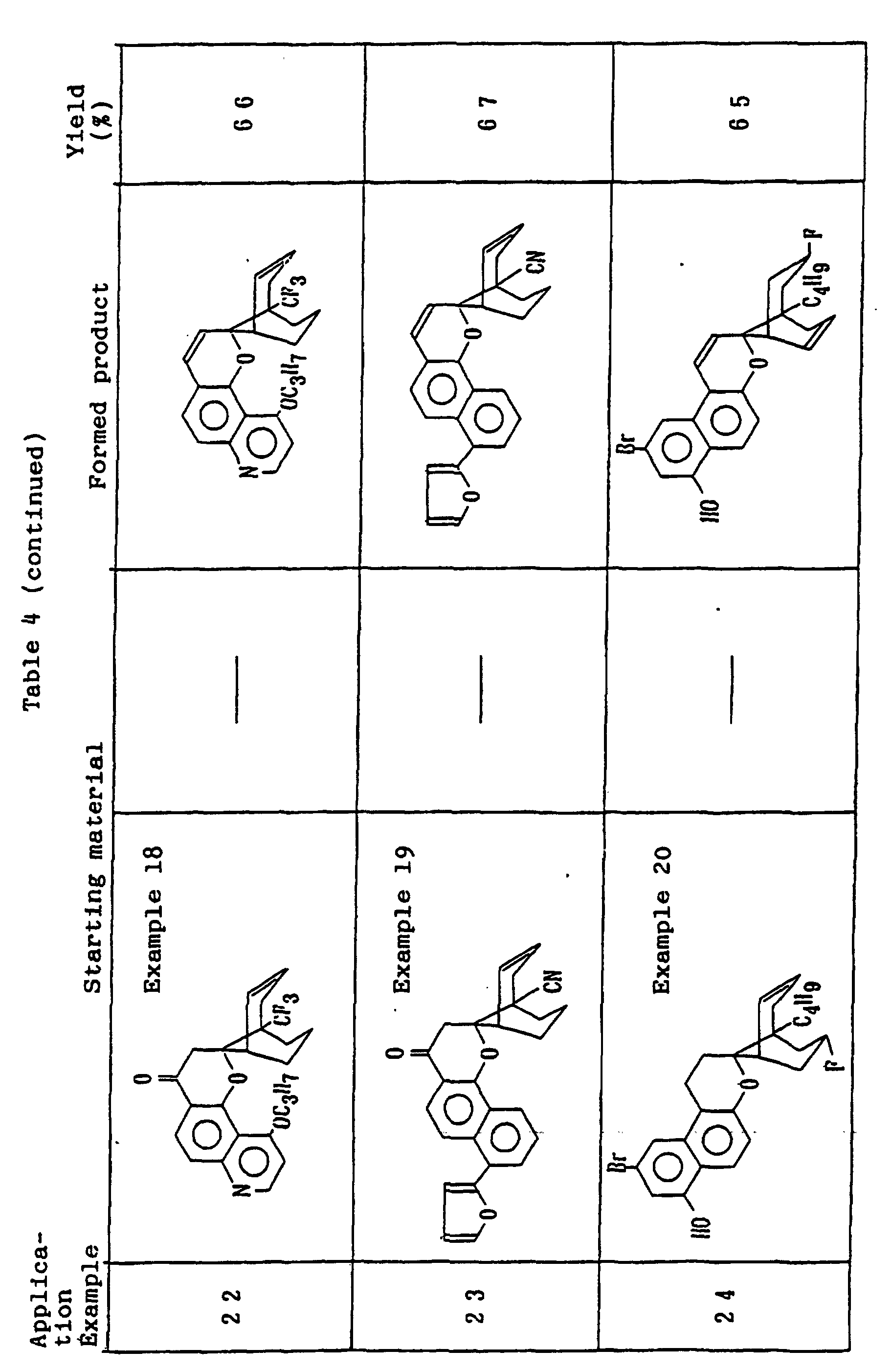

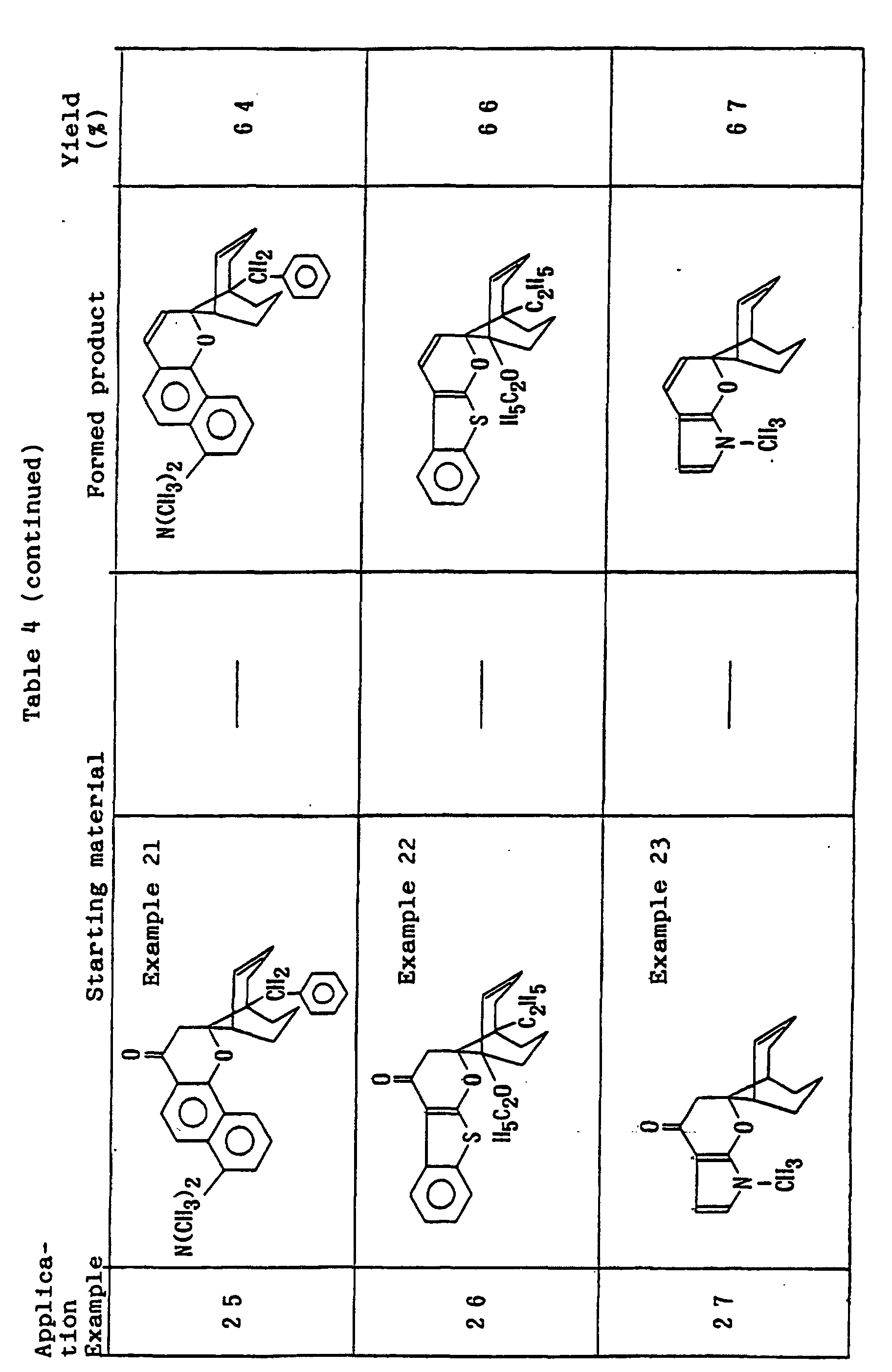

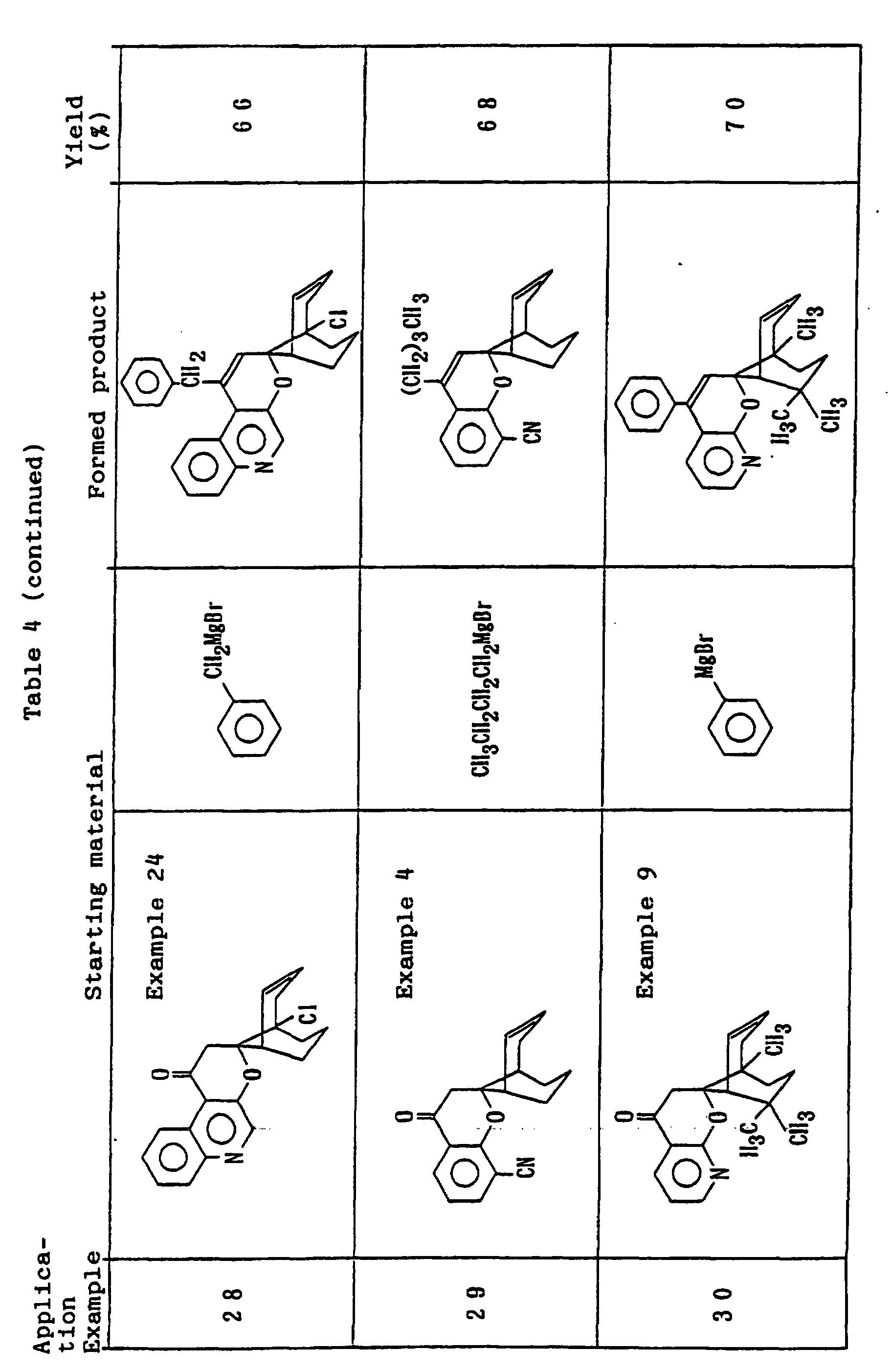

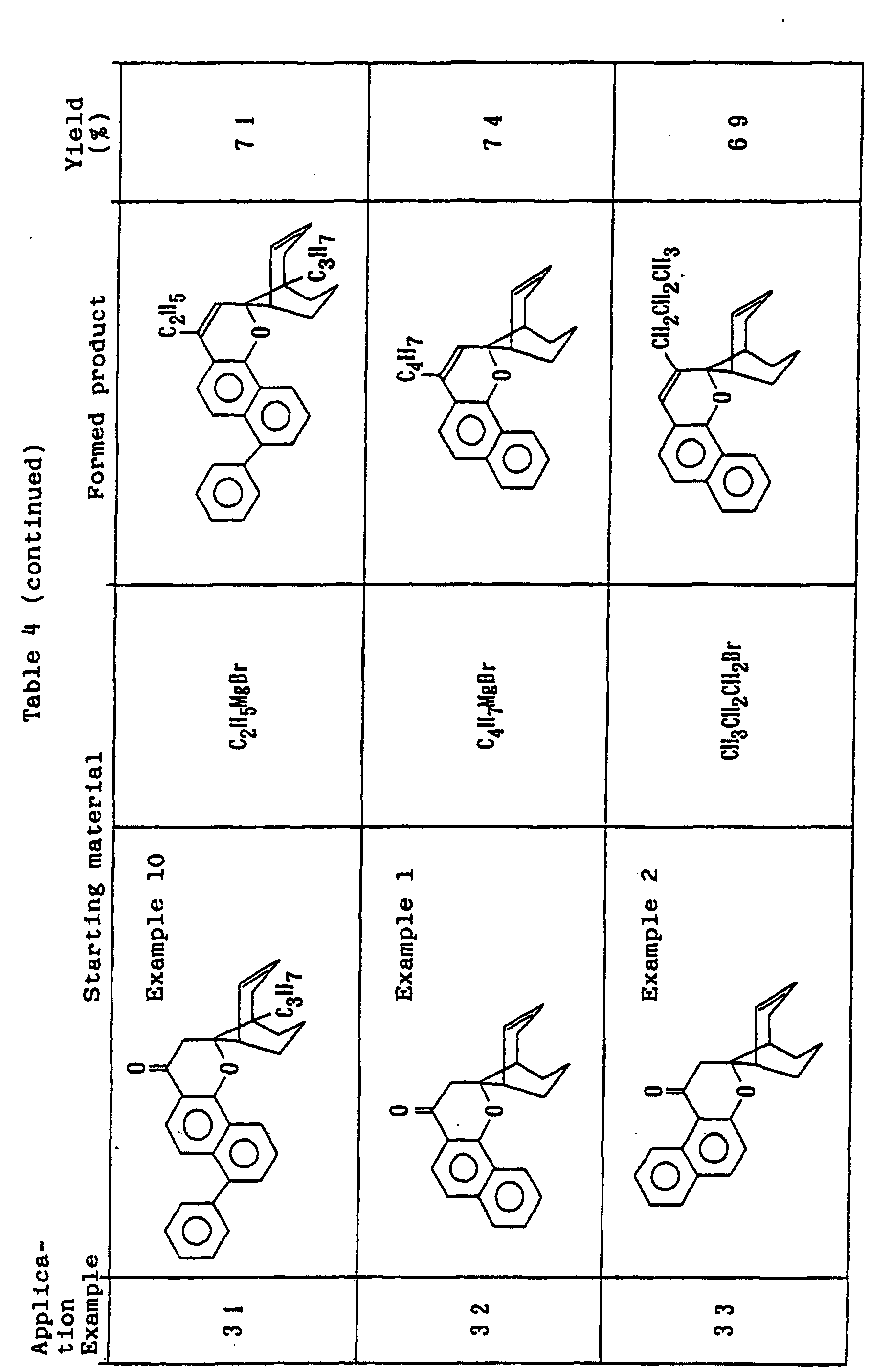

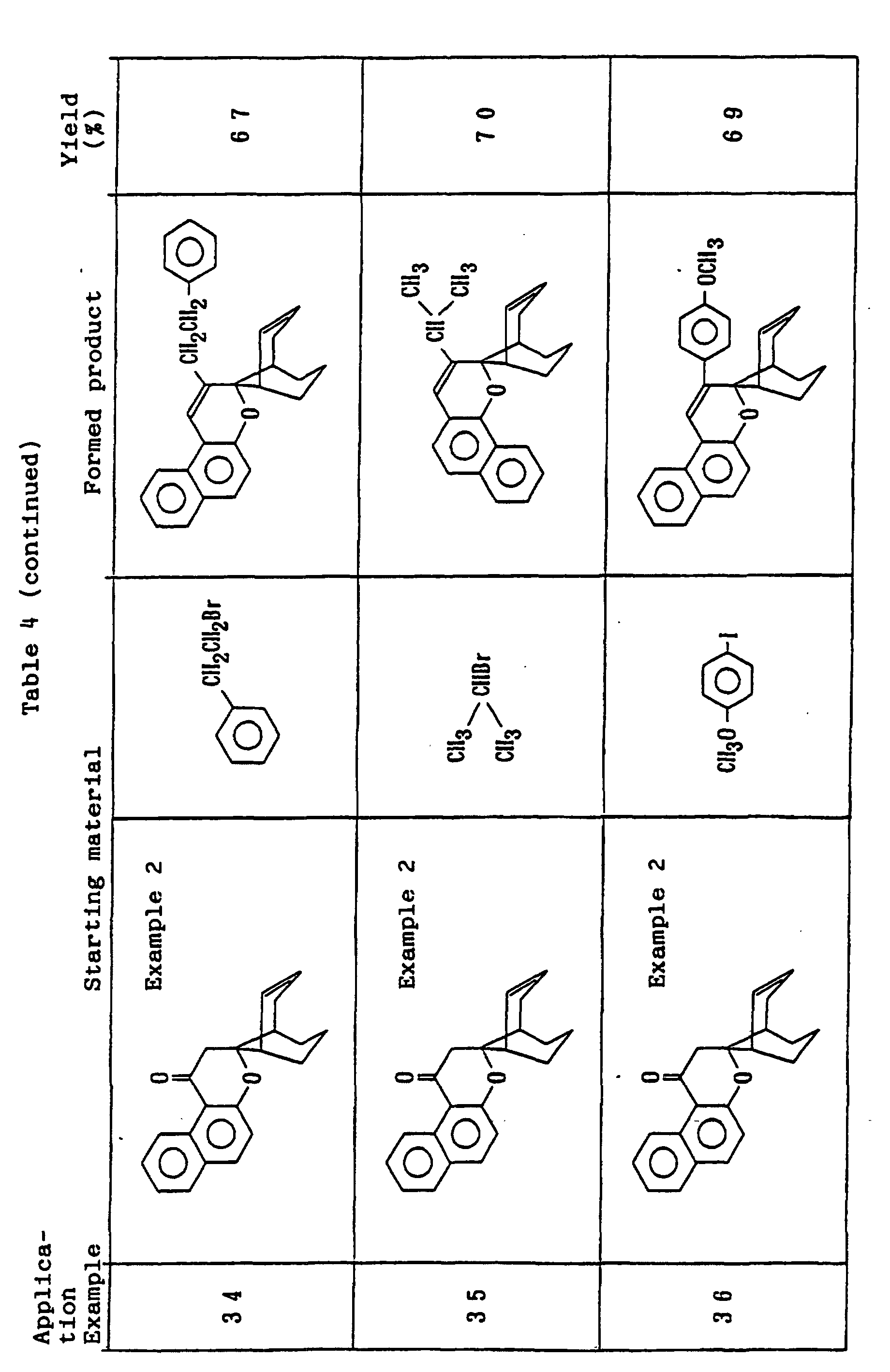

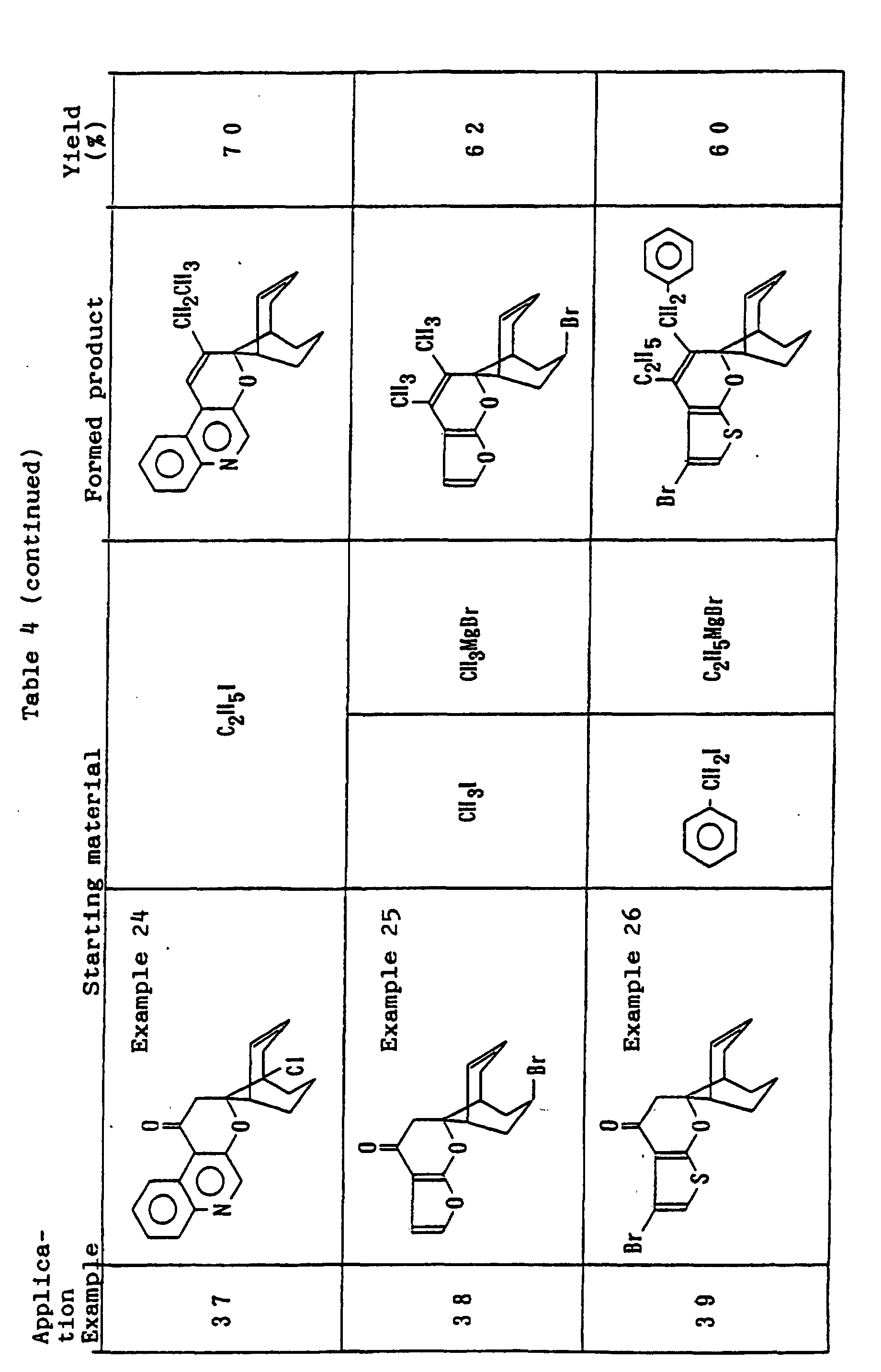

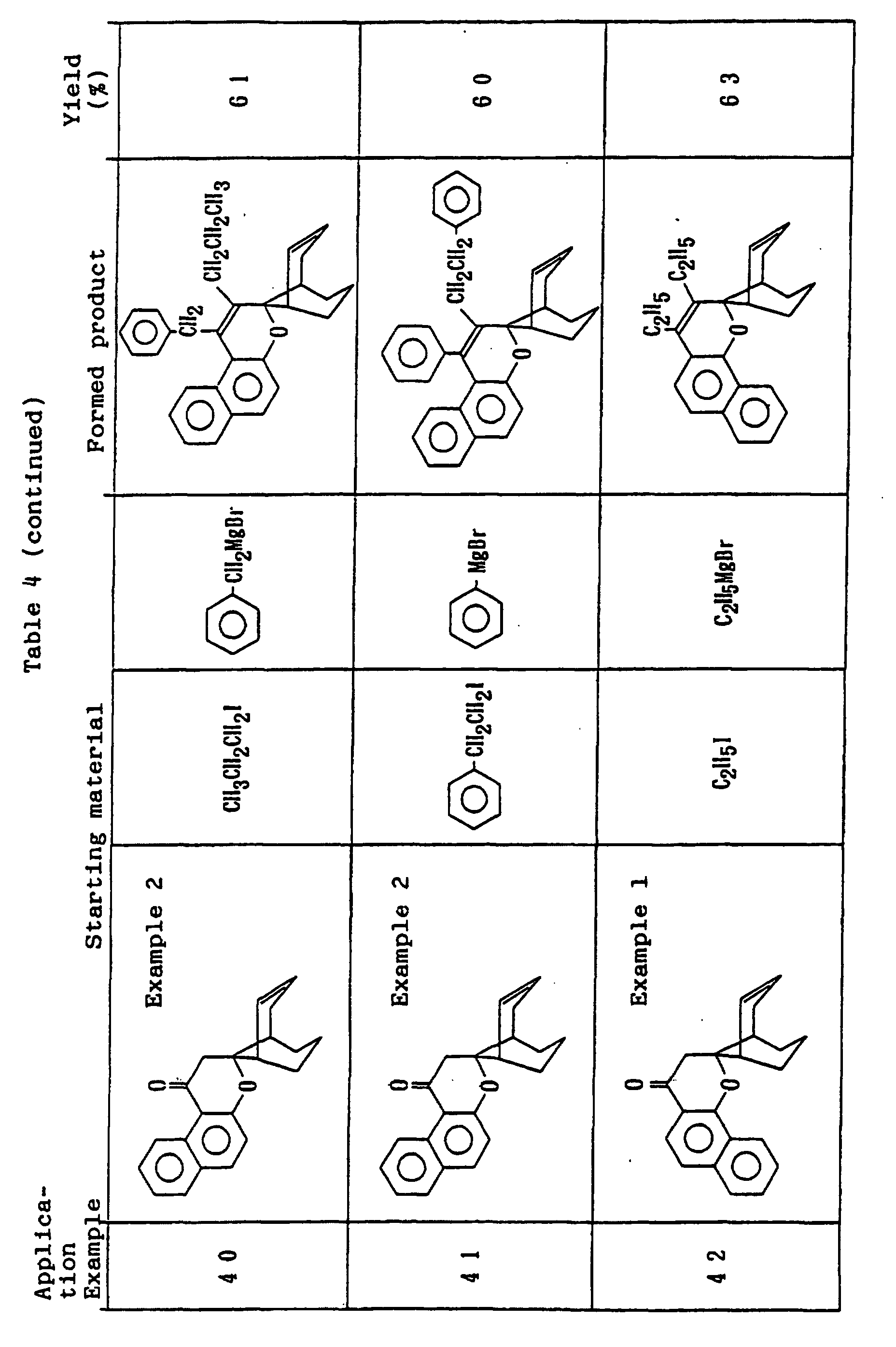

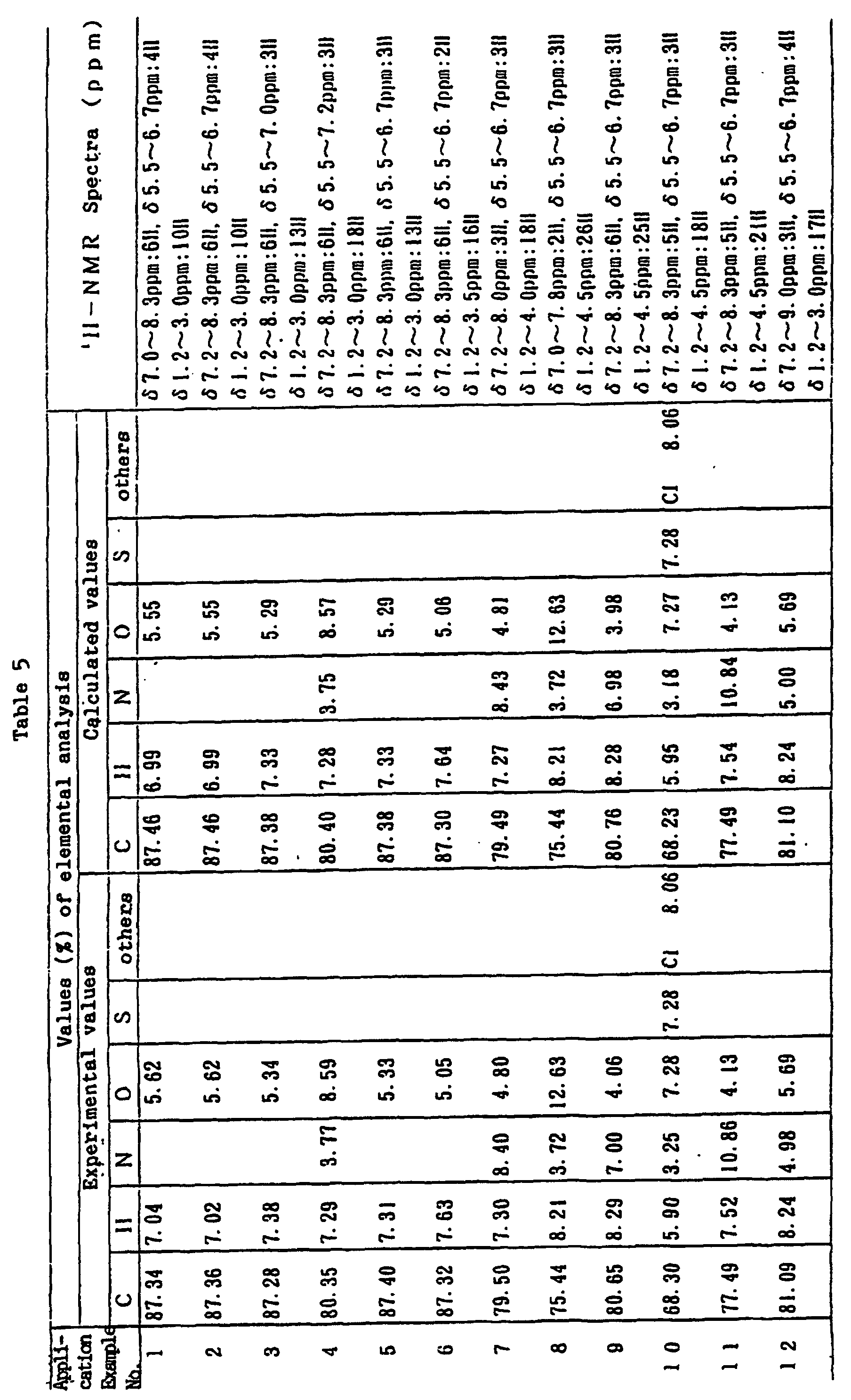

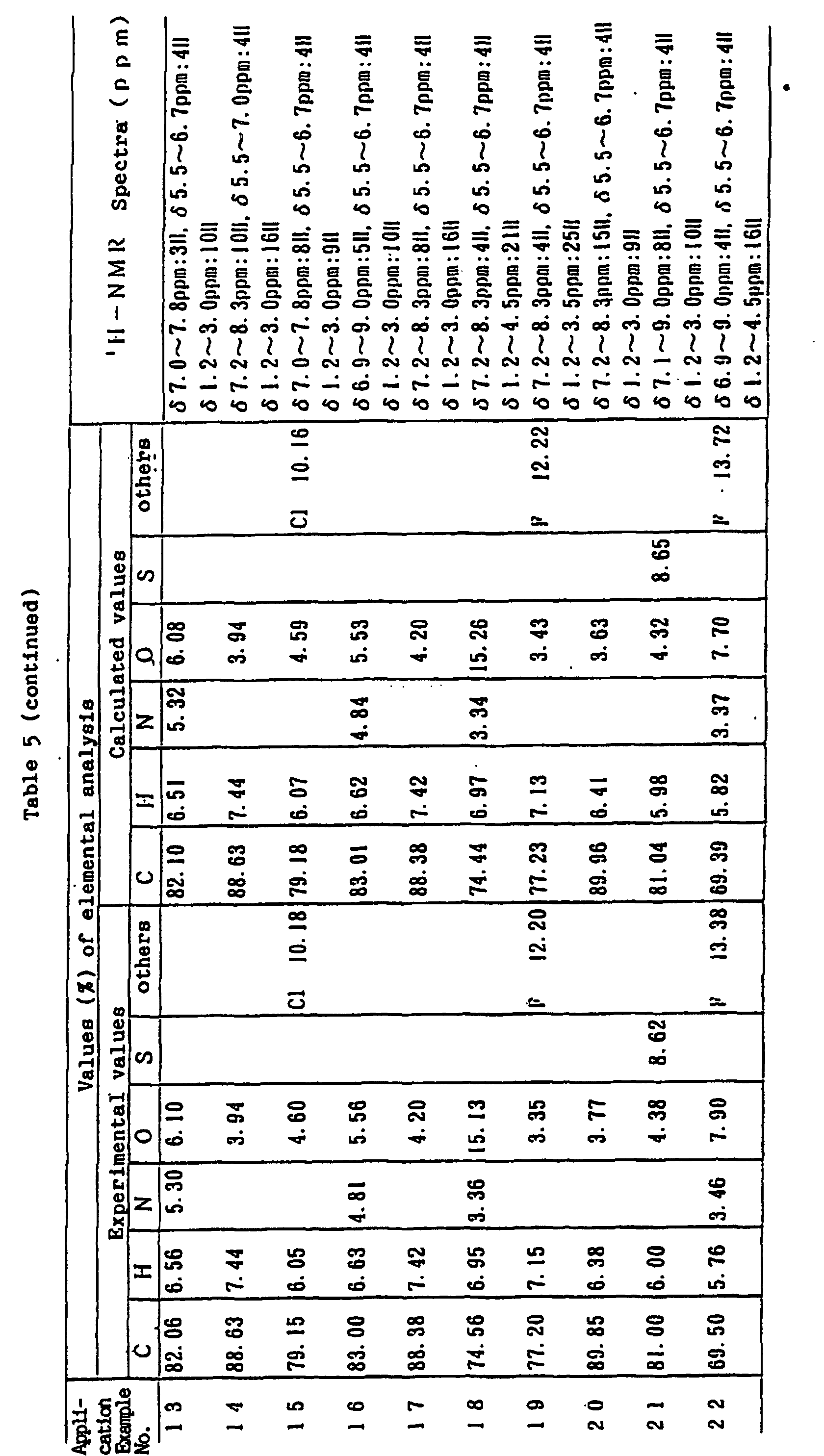

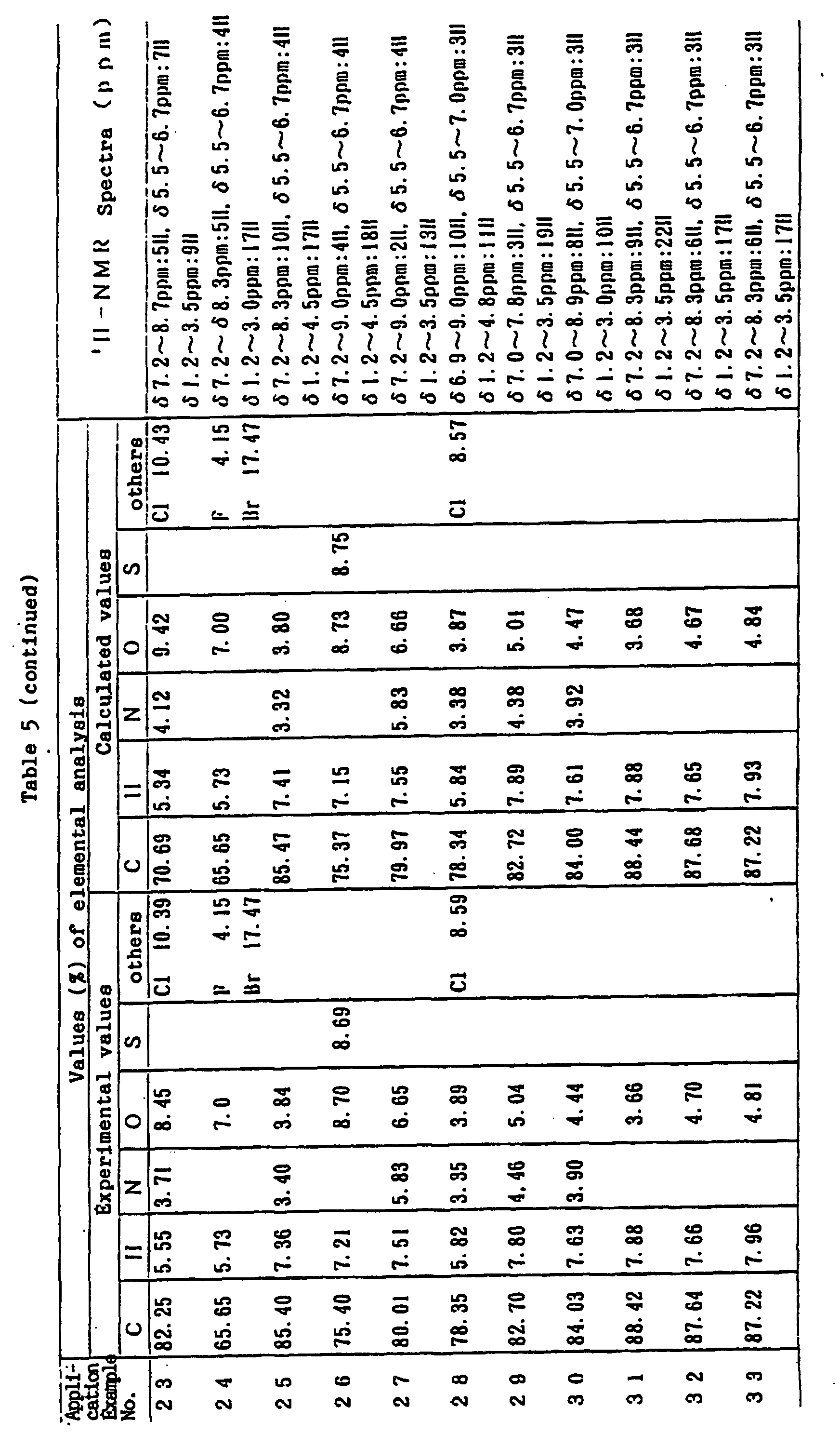

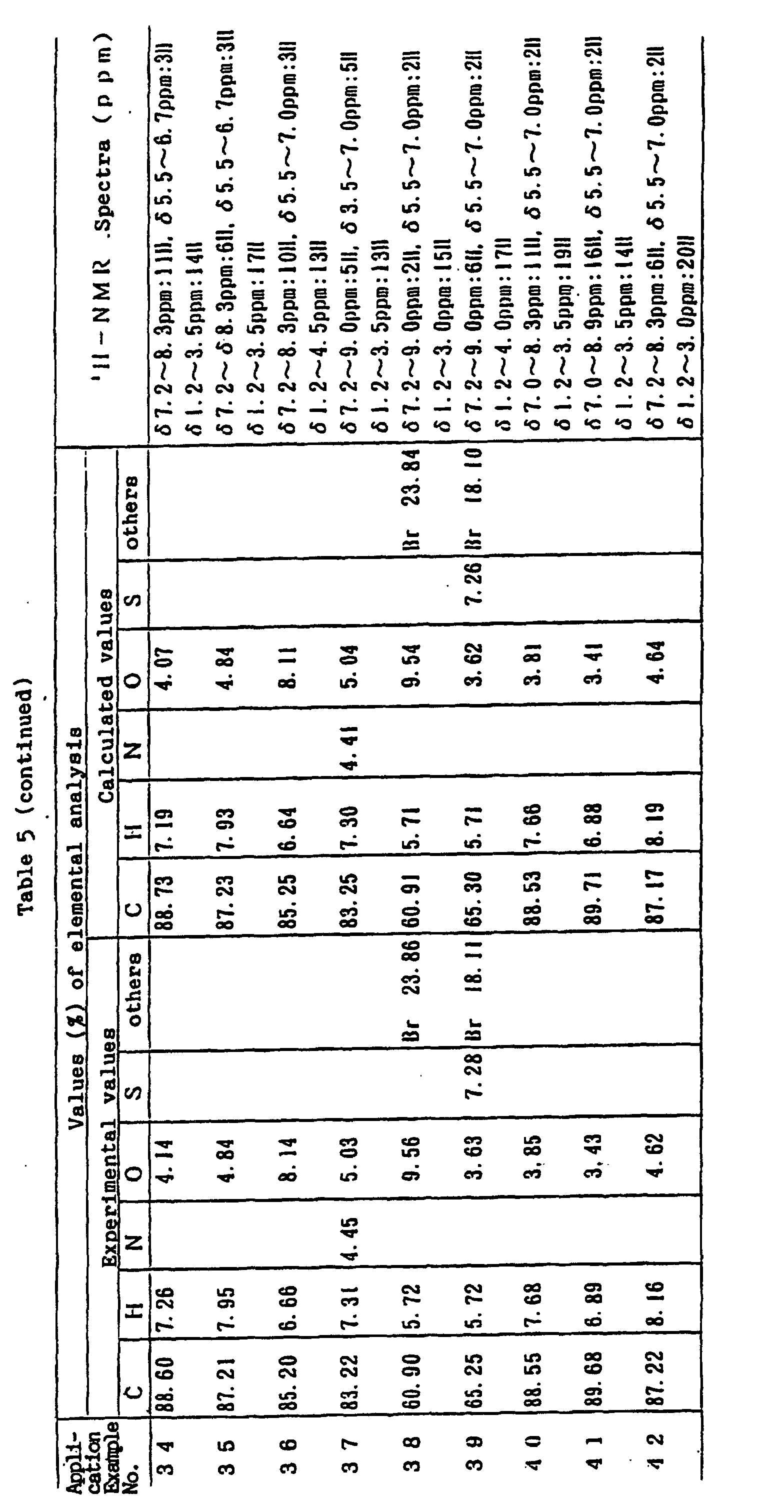

### Application Example 43

The spiropyran compound of the following formula synthesized in Application Example 1 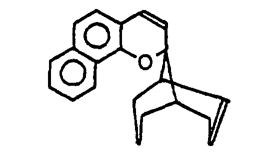 was dissolved and decomposed in a methyl polymethacrylate using benzene, and was casted onto a slide glass (11.2 x 3.7 cm) to form a film thereon. The concentration of the above compound contained in the film was adjusted to be 1.0 x 10⁻⁴ mol/g and the thickness was adjusted to be 0.1 mm. The photochromic film was exposed to the light of a mercury lamp SHL-100 produced by Toshiba Co. at a temperature of 25°C + 1°C from a distance of 10 cm for 60 seconds so as to develop a color, in order to measure its photochromic properties. The photochromic properties were expressed as described below. The results were as shown in Table 6.

| | |
|---|---|
| Max. absorption wavelength (λmax) | λmax of the color-developing film was found by using a spectrophotometer 220A manufactured by Hitachi, Ltd. |
| ε (60 seconds) | Absorbancy of the film at a maximum absorption wavelength after irradiated with light for 60 seconds under the above-mentioned conditions. |
| ε(0 second) | Absorbancy of the film that is not irradiated with light at a maximum absorption wave of when it is irradiated with light. |
| Half-value period t^{1/2} | Time needed until the absorbancy of the film decreases |
| | to one-half {ε(60 sec) - ε(0 sec)} after irradiated with light for 60 seconds. |

### Application Examples 43 to 84

Compounds prepared in Application Examples 2 to 42 were measured for their photochromic properties in the same manner as in Application Example 1. The results were as shown in Table 6.

**Table 6**

| Application Example | Spiropyran compound | Developed color tone | ε (60 sec.) - ε (0 sec.) | λ MAX (nm) | t1/2 (sec.) |
|---|---|---|---|---|---|
| 43 | 1 | yellow | 1.0 | 450 | 115 |
| 44 | 2 | yellow | 0.9 | 403 | 58 |
| 45 | 3 | yellow | 0.7 | 454 | 36 |
| 46 | 4 | yellow | 1.0 | 408 | 185 |
| 47 | 5 | yellow | 0.9 | 454 | 36 |
| 48 | 6 | yellow | 0.8 | 450 | 30 |
| 49 | 7 | orange | 0.7 | 476 | 125 |
| 50 | 8 | orange | 0.8 | 483 | 127 |
| 51 | 9 | yellow | 1.0 | 408 | 180 |
| 52 | 10 | yellow | 1.0 | 410 | 140 |
| 53 | 11 | yellow | 1.2 | 442 | 175 |
| 54 | 12 | yellow | 0.8 | 450 | 50 |
| 55 | 13 | orange | 1.2 | 478 | 110 |
| 56 | 14 | yellow | 1.0 | 440 | 118 |
| 57 | 15 | orange | 0.6 | 470 | 113 |
| 58 | 16 | yellow | 1.0 | 436 | 142 |
| 59 | 17 | yellow | 1.0 | 456 | 138 |
| 60 | 18 | red | 1.1 | 498 | 110 |
| 61 | 19 | yellow | 1.1 | 450 | 125 |
| 62 | 20 | orange | 1.0 | 460 | 70 |
| 63 | 21 | red | 1.1 | 490 | 107 |
| 64 | 22 | orange | 0.9 | 472 | 55 |
| 65 | 23 | red | 1.0 | 505 | 103 |
| 66 | 24 | yellow | 1.0 | 430 | 62 |
| 67 | 25 | red | 1.0 | 486 | 106 |
| 68 | 26 | yellow | 0.9 | 448 | 98 |
| 69 | 27 | orange | 1.0 | 470 | 48 |
| 70 | 28 | yellow | 0.9 | 402 | 16 |
| 71 | 29 | orange | 0.5 | 494 | 11 |
| 72 | 30 | orange | 0.6 | 480 | 26 |
| 73 | 31 | yellow | 0.7 | 460 | 90 |
| 74 | 32 | yellow | 0.8 | 445 | 110 |
| 75 | 33 | yellow | 0.6 | 410 | 18 |
| 76 | 34 | yellow | 0.7 | 406 | 26 |
| 77 | 35 | yellow | 0.6 | 410 | 18 |
| 78 | 36 | yellow | 0.9 | 420 | 25 |
| 79 | 37 | yellow | 0.5 | 415 | 22 |
| 80 | 38 | yellow | 0.6 | 430 | 10 |
| 81 | 39 | orange | 0.6 | 465 | 12 |
| 82 | 40 | yellow | 0.8 | 420 | 14 |
| 83 | 41 | yellow | 0.7 | 440 | 17 |
| 84 | 42 | yellow | 1.0 | 450 | 28 |

## Claims

1. A spiropyrone compound of formula (III) 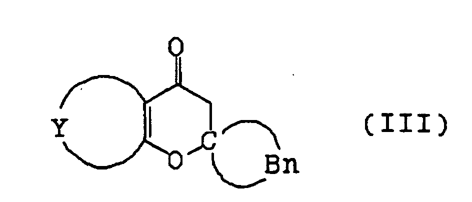 wherein 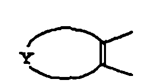 is a substituted or unsubstituted aromatic hydrocarbon group or a substituted or unsubstituted unsaturated heterocyclic group, and 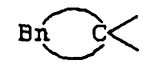 is a substituted or unsubstituted 2-bicyclo[3,3,1]9-nonenylidene group.

2. A compound according to claim 1, wherein 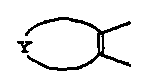 is a divalent aromatic hydrocarbon group or a divalent unsaturated heterocyclic group which is unsubstituted or substituted by one or more substituents selected from a halogen atom, a hydroxyl group, a cyano group, a nitro group, an alkyl group having 1 to 4 carbon atoms, an alkoxy group having 1 to 4 carbon atoms, an aryl group having 6 to 10 carbon atoms, an alkylamino group having 1 to 4 carbon atoms, a dialkylamino group having 2 to 8 carbon atoms, a halogenoalkyl group having 1 to 2 carbon atoms and 5-membered and 6-membered monocyclic heterocyclic groups having one or two sulfur, oxygen or nitrogen atoms.

3. A compound according to claim 2, wherein the number of the said substituents is from 1 to 3.

4. A compound according to claim 2 or 3, wherein the divalent aromatic hydrocarbon group is a benzene ring system or a fused ring system comprising 2 to 4 benzene rings.

5. A compound according to claim 4, wherein the divalent aromatic hydrocarbon group is a benzene, naphthalene or phenanthrene ring system.

6. A compound according to claim 2, wherein the divalent unsaturated heterocyclic group is a 5- or 6-membered monocyclic heterocyclic ring system containing one or two sulfur, oxygen or nitrogen atoms or is a fused heterocyclic ring system in which a benzene ring system is fused to a said monocyclic heterocyclic ring.

7. A compound according to claim 6, wherein the unsaturated heterocyclic group is a pyridine, quinoline, pyrrole, furan, benzofuran, thiophene or benzothiophene ring system.

8. A compound according to any one of the preceding claims, wherein 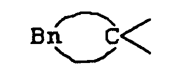 is a bicyclo[3,3,1]9-nonenylidene group, unsubstituted or substituted by one or more substituents selected from a halogen atom, a hydroxyl group, a cyano group, a nitro group, a carboxyl group, an alkyl group having 1 to 4 carbon atoms, an alkoxy group having 1 to 4 carbon atoms, a halogenoalkyl group having 1 to 2 carbon atoms, an aryl group having 6 to 10 carbon atoms, an aryloxy group having 6 to 10 carbon atoms, an aralkyl group having 7 to 10 carbon atoms, an aralkoxy group having 7 to 10 carbon atoms, an alkylamino group having 1 to 4 carbon atoms, a diaklylamino group having 2 to 10 carbon atoms and an alkoxycarbonyl group having 2 to 10 carbon atoms.

9. A compound according to claim 8, wherein the number of the said substituents is from 1 to 3.

10. A process for preparing a spiropyrone compound of formula (III) according to any one of the preceding claims, which process comprises reacting a compound of formula (I) 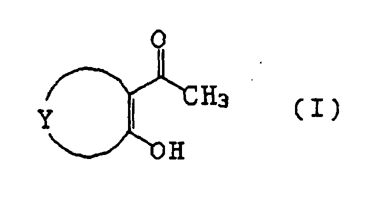 wherein 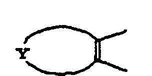 is as defined in any one of claims 1 to 7, with a compound of formula (II) 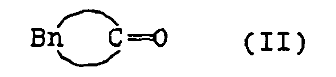 wherein 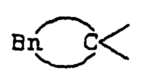 is as defined in any one of claims 1, 8 and 9.

11. A process according to claim 10, wherein the reaction is carried out in the presence of a condensing agent.

12. A process according to claim 11, wherein the condensing agent is a primary amine or a secondary amine.

13. A process for preparing a spiropyran compound of formula (IV) 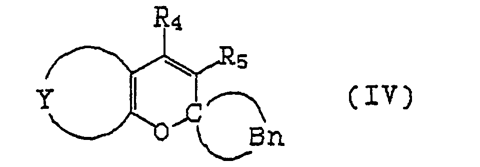 wherein 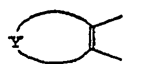 is as defined in any one of claims 1 to 7, 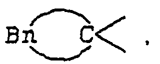 is as defined in any one of claims 1, 8 or 9 and R₄ and R₅, which are the same or different, are selected from a hydrogen atom and alkyl, aryl, aralkyl and substituted amino groups with the proviso that, when either R₄ or R₅ is a substituted amino group, the other is a hydrogen atom which process comprises:
(a) reducing a compound of formula (III) as defined in any one of claims 1 to 9 and dehydrating a thus obtained compound of formula (XI) 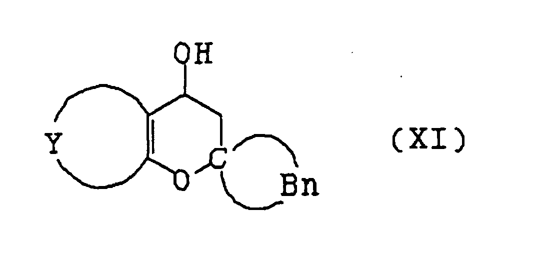 thereby to obtain a said spiropyran compound of formula (IV) is which both R₄ and R₅ are hydrogen; or
(b) reacting a compound of formula (III) as defined in any one of claims 1 to 9 with a compound of formula
R-MgX
or
R-Li
wherein R is an alkyl group, an aryl group or an aralkyl group and X is a halogen atom,
and dehydrating a thus obtained compound of formula (XII) 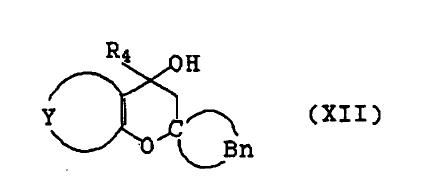 thereby to obtain a compound of formula (IV) in which R₅ is hydrogen; or
(c) reacting a compound of formula (III) as defined in any one of claims 1 to 9 with an amine of formula
R-H
wherein R is a substituted amino group, thereby to obtain a compound of formula (IV) in which one of R₄ and R₅ is a substituted amino group and the other is hydrogen; or
(d) reacting a compound of formula (IV) in which one of R₄ and R₅ is a substituted amino group and the other is hydrogen with a compound of the formula
R₅X
wherein R₅ is an alkyl, an aralkyl or an aryl group,
and reducing and dehydrating a thus obtained compound of formula (XIII) 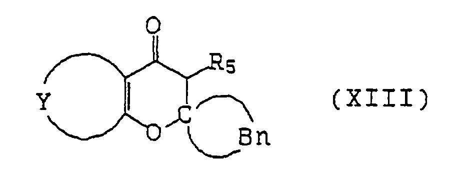 thereby to obtain a compound of formula (IV) in which R₄ is hydrogen; or
(e) reacting a compound of formula (XIII) 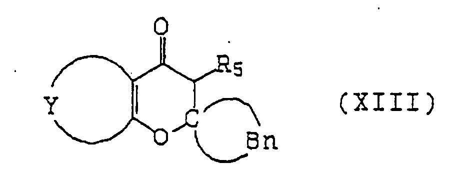 with a compound of the formula
R₄-MgX
or
R₄-Li
wherein R₄ and R₅ are the same or different alkyl groups, aralkyl groups or aryl groups,
and dehydrating a thus obtained compound of formula (XII) 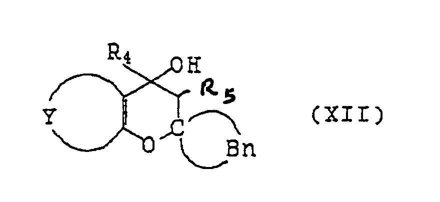 thereby to obtain a compound of formula (IV).

## Patentansprüche

1. Spiropyron-Verbindung der Formel (III) 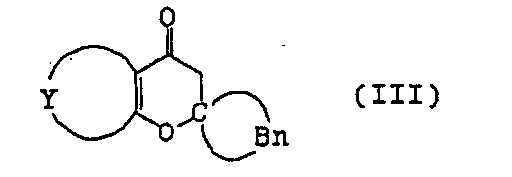 worin 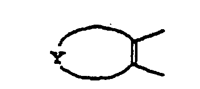
eine substituierte oder unsubstituierte aromatische Kohlenwasserstoffgruppe oder eine substituierte oder unsubstituierte ungesättigte heterocyclische Gruppe bedeutet und 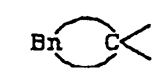
eine substituierte oder unsubstituierte 2-Bicyclo[3.3.1]-9-nonenylidengruppe bedeutet.

2. Verbindung nach Anspruch 1, wobei 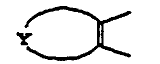 eine zweiwertige aromatische Kohlenwasserstoffgruppe oder eine zweiwertige ungesättigte heterocyclische Gruppe bedeutet, die unsubstituiert oder durch einen oder mehrere Substituenten, die unter Halogenatomen, Hydroxylgruppen, Cyanogruppen, Nitrogruppen, Alkylgruppen mit 1 bis 4 Kohlenstoffatomen, Alkoxygruppen mit 1 bis 4 Kohlenstoffatomen, Arylgruppen mit 6 bis 10 Kohlenstoffatomen, Alkylaminogruppen mit 1 bis 4 Kohlenstoffatomen, Dialkylaminogruppen mit 2 bis 8 Kohlenstoffatomen, Halogenalkylgruppen mit 1 oder 2 Kohlenstoffatomen und 5-gliedrigen und 6-gliedrigen monocyclischen heterocyclischen Gruppen mit 1 oder 2 Schwefel-, Sauerstoff- oder Stickstoffatomen ausgewählt sind, substituiert ist.

3. Verbindung nach Anspruch 2, wobei die Anzahl der Substituenten 1 bis 3 beträgt.

4. Verbindung nach Anspruch 2 oder 3, wobei es sich bei der zweiwertigen aromatischen Kohlenwasserstoffgruppe um ein Benzolringsystem oder ein kondensiertes Ringsystem mit 2 bis 4 Benzolringen handelt.

5. Verbindung nach Anspruch 4, wobei es sich bei der zweiwertigen aromatischen Kohlenwasserstoffgruppe um ein Benzol-, Naphthalin- oder Phenantren-Ringsystem handelt.

6. Verbindung nach Anspruch 2, wobei es sich bei der zweiwertigen ungesättigten heterocyclischen Gruppe um ein 5- oder 6-gliedriges monocyclisches heterocyclisches Ringsystem mit einem oder zwei Schwefel-, Sauerstoff- oder Stickstoffatomen oder um ein kondensiertes heterocyclisches Ringsystem, bei dem ein Benzolringsystem an den monocyclischen heterocyclischen Ring kondensiert ist, handelt.

7. Verbindung nach Anspruch 6, wobei es sich bei der ungesättigten heterocyclischen Gruppe um ein Pyridin-, Chinolin-, Pyrrol-, Furan-, Benzofuran-, Thiophen- oder Benzothiophen-Ringsystem handelt.

8. Verbindung nach einem der vorstehenden Ansprüche, wobei 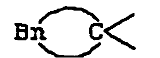 eine Bicyclo[3.3.1]-9-nonenylidengruppe bedeutet, die unsubstituiert oder durch einen oder mehrere Substituenten substituiert ist, die unter Halogenatomen, Hydroxylgruppen, Cyanogruppen, Nitrogruppen, Carboxylgruppen, Alkylgruppen mit 1 bis 4 Kohlenstoffatomen, Alkoxygruppen mit 1 bis 4 Kohlenstoffatomen, Halogenalkylgruppen mit 1 oder 2 Kohlenstoffatomen, Arylgruppen mit 6 bis 10 Kohlenstoffatomen, Aryloxygruppen mit 6 bis 10 Kohlenstoffatomen, Aralkylgruppen mit 7 bis 10 Kohlenstoffatomen, Aralkoxygruppen mit 7 bis 10 Kohlenstoffatomen, Alkylaminogruppen mit 1 bis 4 Kohlenstoffatomen, Dialkylaminogruppen mit 2 bis 10 Kohlenstoffatomen und Alkoxycarbonylgruppen mit 2 bis 10 Kohlenstoffatomen ausgewählt sind.

9. Verbindung nach Anspruch 8, wobei die Anzahl der Substituenten 1 bis 3 beträgt.

10. Verfahren zur Herstellung einer Spiropyron-Verbindung der Formel (III) nach einem der vorstehenden Ansprüche, wobei das Verfahren die Umsetzung einer Verbindung der Formel (I) 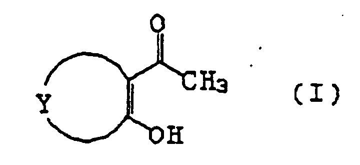 worin 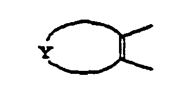
der Definition in einem der Ansprüche 1 bis 7 entspricht, mit einer Verbindung der Formel (II) 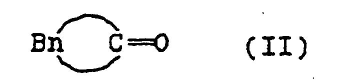 worin 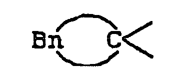
der Definition in einem der Ansprüche 1, 8 und 9 entspricht, umfasst.

11. Verfahren nach Anspruch 10, wobei die Umsetzung in Gegenwart eines Kondensationsmittels durchgeführt wird.

12. Verfahren nach Anspruch 11, wobei es sich beim Kondensationsmittel um ein primäres Amin oder um ein sekundäres Amin handelt.

13. Verfahren zur Herstellung einer Spiropyran-Verbindung der Formel (IV) 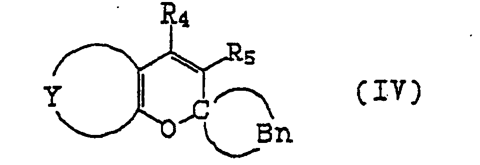 worin 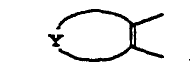
der Definition in einem der Ansprüche 1 bis 7 entspricht, 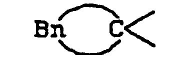
der Definition in einem der Ansprüche 1, 8 oder 9 entspricht und R₄ und R₅, die gleich oder verschieden sind, unter Wasserstoffatomen, Alkylgruppen, Arylgruppen, Aralkylgruppen und substituierten Aminogruppen ausgewählt sind, mit der Maßgabe, dass dann, wenn es sich bei einem der Reste R₄ oder R₅ um eine substituierte Aminogruppe handelt, der andere Rest ein Wasserstoffatom bedeutet,
wobei das Verfahren folgendes umfasst:
(a) Reduzieren einer Verbindung der Formel (III) gemäß der Definition in einem der Ansprüche 1 bis 9 und Dehydratisieren der auf diese Weise erhaltenen Verbindung der Formel (XI) 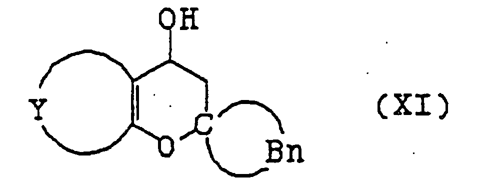 unter Bildung der Spiropyran-Verbindung der Formel (IV), in der die beiden Reste R₄ und R₅ Wasserstoff bedeuten; oder
(b) Umsetzen einer Verbindung der Formel (III) gemäß der Definition in einem der Ansprüche 1 bis 9 mit einer Verbindung der Formel
R-MgX
oder
R-Li
worin R eine Alkylgruppe, eine Arylgruppe oder eine Aralklygruppe bedeutet und X ein Halogenatom bedeutet,
und Dehydratisieren der auf diese Weise erhaltenen Verbindung der Formel (XII) 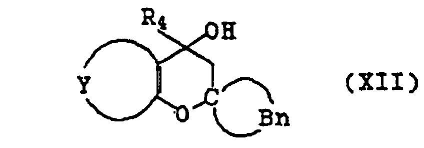 unter Bildung einer Verbindung der Formel (IV), in der R₅ Wasserstoff bedeutet; oder
(c) Umsetzen einer Verbindung der Formel (III) gemäß der Definition in einem der Ansprüche 1 bis 9 mit einem Amin der Formel
R-H
worin R eine substituierte Aminogruppe bedeutet, unter Bildung einer Verbindung der Formel (IV), in der einer der Reste R₄ und R₅ eine substituierte Aminogruppe bedeutet und der andere Wasserstoff bedeutet; oder
(d) Umsetzen einer Verbindung der Formel (IV), in der einer der Reste R₄ und R₅ eine substituierte Aminogruppe bedeutet und der andere Wasserstoff bedeutet, mit einer Verbindung der Formel
R₅X,
worin R₅ eine Alkylgruppe, eine Aralkylgruppe oder eine Arylgruppe bedeutet,
und Reduzieren und Dehydratisieren der auf diese Weise erhaltenen Verbindung der Formel (XIII) 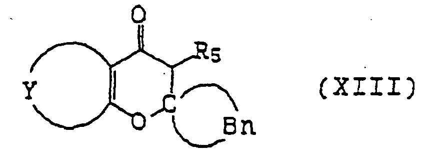 unter Bildung einer Verbindung der Formel (IV), in der R₄ Wasserstoff bedeutet; oder
(e) Umsetzen einer Verbindung der Formel (XIII) 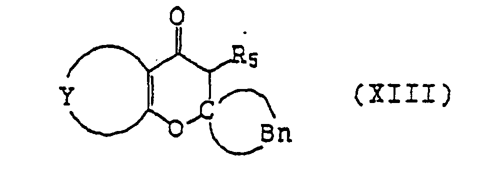 mit einer Verbindung der Formel
R₄-MgX
oder
R₄-Li
worin R₄ und R₅ gleiche oder verschiedene Alkylgruppen, Aralkylgruppen oder Arylgruppen bedeuten,
und Dehydratisieren der auf diese Weise erhaltenen Verbindung der Formel (XII) 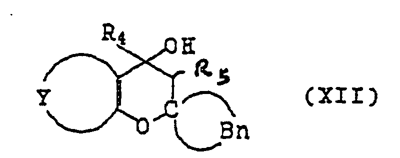 unter Bildung einer Verbindung der Formel (IV).

## Revendications

1. Composé de spiropyrone ayant la formule 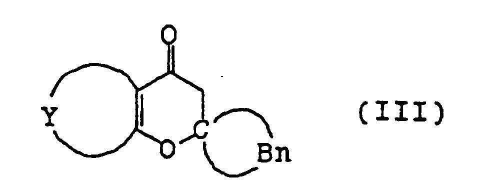 (III)
où 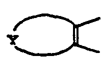 est un groupe hydrocarbure aromatique substitué ou non-substitué ou un groupe hétérocyclique non-saturé substitué ou non-substitué, et 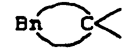 est un groupe 2-bicyclo[3,3,1]9-nonenylidène substitué ou non-substitué.

2. Composé selon la revendication 1, dans lequel 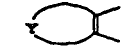 est un groupe hydrocarbure aromatique divalent ou un groupe hétérocyclique non-saturé divalent qui est non-substitué ou substitué par un ou plusieurs substituants sélectionnés parmi un atome d'halogène, un groupe hydroxyle, un groupe cyano, un groupe nitro, un groupe alkyle ayant 1 à 4 atomes de carbone, un groupe alkoxy ayant 1 à 4 atomes de carbone, un groupe aryle ayant 6 à 10 atomes de carbone, un groupe alkylamino ayant 1 à 4 atomes de carbone, un groupe dialkylamino ayant 2 à 8 atomes de carbone, un groupe halogénoalkyle ayant 1 à 2 atomes de carbone, et des groupes hétérocycliques monocycliques à 5 éléments et 6 éléments ayant un ou deux atomes de soufre, d'oxygène ou d'azote.

3. Composé selon la revendication 2, dans lequel le nombre desdits substituants est compris entre 1 et 3.

4. Composé selon la revendication 2 ou 3, dans lequel le groupe hydrocarbure aromatique divalent est un système d'anneaux benzène ou un système d'anneaux fusionnés comportant 2 à 4 anneaux benzène.

5. Composé selon la revendication 4, dans lequel le groupe hydrocarbure aromatique divalent est un système d'anneaux benzène, naphtalène ou phénanthrène.

6. Composé selon la revendication 2, dans lequel le groupe hétérocyclique non-saturé divalent est un système d'anneaux hétérocycliques monocycliques à 5 ou 6 éléments contenant un ou deux atomes de soufre, d'oxygène
ou d'azote ou est un système d'anneaux hétérocycliques fusionnés dans lequel un système d'anneaux benzène est fusionné audit anneau hétérocyclique monocyclique.

7. Composé selon la revendication 6, dans lequel le groupe hétérocyclique non-saturé est un système d'anneaux de pyridine, quinoline, pyrrole, furanne, benzofuranne, thiophène ou benzothiophène.

8. Composé selon l'une quelconque des revendications précédentes, dans lequel 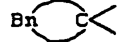 est un groupe bicyclo[3,3,1]9-nonenylidène, non-substitué ou substitué par un ou plusieurs substituants sélectionnés parmi un atome d'halogène, un groupe hydroxyle, un groupe cyano, un groupe nitro, un groupe carboxyle, un groupe alkyle ayant 1 à 4 atomes de carbone, un groupe alkoxy ayant 1 à 4 atomes de carbone, un groupe halogénoalkyle ayant 1 à 2 atomes de carbone, un groupe aryle ayant 6 à 10 atomes de carbone, un groupe aryloxy ayant 6 à 10 atomes de carbone, un groupe aralkyle ayant 7 à 10 atomes de carbone, un groupe aralkoxy ayant 7 à 10 atomes de carbone, un groupe alkylamino ayant 1 à 4 atomes de carbone, un groupe dialkylamino ayant 2 à 10 atomes de carbone et un groupe alkoxycarbonyle ayant 2 à 10 atomes de carbone.

9. Composé selon la revendication 8, dans lequel le nombre desdits substituants est compris entre 1 et 3.

10. Procédé pour préparer un composé de spiropyrone ayant la formule (III) selon l'une quelconque des des revendications précédentes, lequel procédé comporte la réaction d'un composé ayant la formule (I) 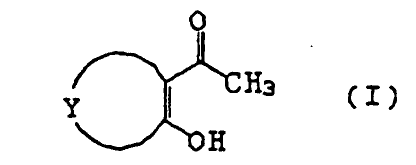 où 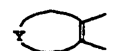 est comme défini selon l'une quelconque des revendications 1 à 7, avec un composé ayant la formule (II) 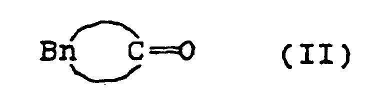 où 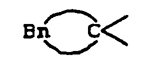 est comme défini selon l'une quelconque des revendications 1, 8 et 9.

11. Procédé selon la revendication 10, dans lequel la réaction est effectuée en présence d'un agent de condensation.

12. Procédé selon la revendication 11, dans lequel l'agent de condensation est une amine primaire ou une amine secondaire.

13. Procédé pour préparer un composé de spiropyrane ayant la formule (IV) 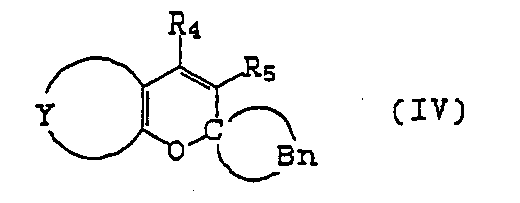 où 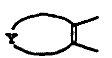 est comme défini selon l'une des revendications 1 à 7, 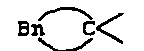 est comme défini selon l'une quelconque des revendications 1, 8 et 9 et R₄ et R₅, qui sont identiques ou différents, sont sélectionnés parmi un atome d'hydrogène et des groupes alkyle, aryle, aralkyle et amino substitué pourvu que, lorsque R₄ ou R₅ est un groupe amino substitué, l'autre est un atome d'hydrogène, lequel procédé comporte les étapes consistant à :
(a) réduire un composé ayant la formule (III) comme défini selon l'une quelconque des revendications 1 à 9 et déshydrater un composé ainsi obtenu ayant la formule (XI) 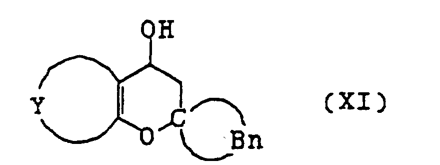 de manière à obtenir ledit composé de spiropyrane ayant la formule (IV) où R₄ et R₅ sont tous deux de l'hydrogène, ou
(b) faire réagir un composé ayant la formule (III) comme défini selon l'une quelconque des revendications 1 à 9 avec un composé ayant la formule
R-MgX
ou
R-Li
où R est un groupe alkyle, un groupe aryle ou un groupe aralkyle et X est un atome d'halogène, et déshydrater un composé ainsi obtenu ayant la formule (XII) 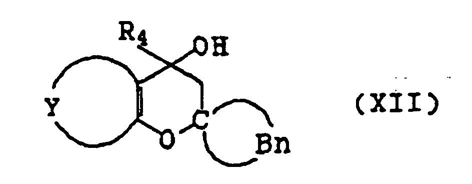 de manière à obtenir un composé ayant la formule (IV) où R₅ est de l'hydrogène, ou
(c) faire réagir un composé ayant la formule (III) comme défini selon l'une quelconque des revendications 1 à 9 avec une amine ayant la formule
R-H
où R est un groupe amino substitué, de manière à obtenir le composé ayant la formule (IV) dans lequel un élément parmi R₄ et R₅ est un groupe amino substitué et l'autre est de l'hydrogène, ou
(d) faire réagir un composé ayant la formule (IV) dans lequel un élément parmi R₄ et R₅ est un groupe amino substitué et l'autre est de l'hydrogène avec un composé ayant la formule
R₅X
où R₅ est un groupe alkyle, aralkyle ou aryle, et réduire et déshydrater un composé ainsi obtenu ayant la formule (XIII) 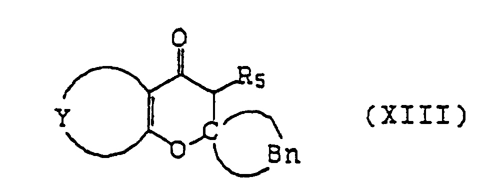 de manière à obtenir un composé ayant la formule (IV) où R₄ est de l'hydrogène, ou
(e) faire réagir un composé ayant la formule (XIII) 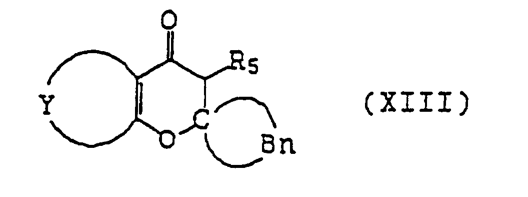 avec un composé ayant la formule
R₄-MgX
ou
R₄-Li
où R₄ et R₅ sont des groupes alkyle, des groupes aralkyle ou des groupes aryle identiques ou différents,
et déshydrater un composé ainsi obtenu ayant la formule (XII) 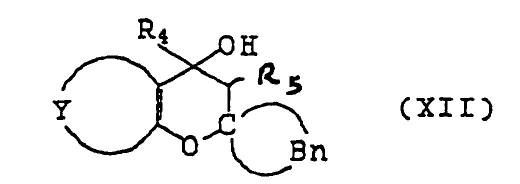 de manière à obtenir un composé ayant la formule (IV).
